(19) Europäisches Patentamt
European Patent Office
Office européen des brevets

(11) EP 3 072 975 A1

(12) **EUROPEAN PATENT APPLICATION**
published in accordance with Art. 153(4) EPC

(43) Date of publication:
**28.09.2016 Bulletin 2016/39**

(21) Application number: **14863863.8**

(22) Date of filing: **21.11.2014**

(51) Int Cl.:
***C12Q 1/26*** *(2006.01)* ***C12Q 1/32*** *(2006.01)*
***C12Q 1/48*** *(2006.01)* ***G01N 21/78*** *(2006.01)*

(86) International application number:
**PCT/JP2014/080865**

(87) International publication number:
**WO 2015/076361 (28.05.2015 Gazette 2015/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.11.2013 JP 2013240926**

(71) Applicant: **The University of Tokyo**
**Tokyo 113-8654 (JP)**

(72) Inventors:
- **KUMAGAI Kazuo**
**Tokyo 113-8654 (JP)**
- **OKABE Takayoshi**
**Tokyo 113-8654 (JP)**
- **KOJIMA Hirotatsu**
**Tokyo 113-8654 (JP)**
- **NAGANO Tetsuo**
**Tokyo 113-8654 (JP)**

(74) Representative: **Desaix, Anne et al**
**Ernest Gutmann - Yves Plasseraud SAS**
**3, rue Auber**
**75009 Paris (FR)**

(54) **METHOD FOR DETECTING FLUORESCENCE OR ABSORBANCE, METHOD FOR SUPPRESSING BACKGROUND, METHOD FOR MEASURING ADP, METHOD FOR MEASURING ACTIVITY OF ADP-SYNTHESIZING ENZYME, AND METHOD FOR MEASURING ACTIVITY OF GLUCOSYLTRANSFERASE**

(57) In a method for detecting fluorescence or absorbance of the present invention, a diaphorase causes reduction from resazurin to resorufin in the presence of an SH reagent and NADH or NADPH, and the resulting fluorescence intensity or absorbance is measured. A method for measuring ADP of the present invention includes a 2-1 process in which glucose is reacted with ADP and an ADP-dependent hexokinase, a 2-2 process in which the glucose-6-phosphate obtained in the 2-1 process is reacted with NAD or NADP and glucose-6-phosphate dehydrogenase, and a 2-3 process in which resazurin is reacted with the NADH or NADPH obtained in the 2-2 process and a diaphorase in the presence of an SH reagent, and the resulting fluorescence intensity or absorbance is measured.

EP 3 072 975 A1

## Description

[Technical Field]

The present invention relates to a method for detecting fluorescence or absorbance through which it is possible to measure a fluorescence intensity or absorbance resulting from reduction of resazurin to resorufin with high sensitivity, a method for suppressing background, a method for measuring ADP, a method for measuring activities of ADP-producing enzymes such as kinases or glycosyltransferases with high sensitivity in a simple and easy manner, a method for screening for activity control agents of glycosyltransferases or ADP-producing enzymes such as kinase using the same, and a measurement kit for the same.

Priority is claimed on Japanese Patent Application No. 2013-240926, filed November 21, 2013, the content of which is incorporated herein by reference.

[Background Art]

"Glycosyltransferase" is a general term for enzymes that transfer a glycosyl group from a donor (G) including the glycosyl group to a receptor (A), and catalyze a reaction connecting G-A (Non-Patent Literature 1). In animals, 9 types of sugar nucleotides (GDP-fucose, GDP-mannose, UDP-glucose, UDP-galactose, UDP-glucuronic acid, UDP-xylose, UDP-N-acetylglucosamine, UDP-N-acetylgalactosamine, and CMP-sialic acid) are main donor molecules (Non-Patent Literature 2).

The glycosyltransferases that transfer glycosyl groups from these donor molecules are called fucosyltransferases, mannosyltransferases, glucosyltransferases, galactosyltransferases, glucuronosyltransferases, xylosyltransferases, N-acetylglucosaminyltransferases, N-acetylgalactosaminyltransferases, and sialyltransferases.

Guanosine 5'-diphosphate is abbreviated as GDP, uridine 5'-diphosphate is abbreviated as UDP, and cytidine 5'-monophosphate is abbreviated as CMP. Exemplary receptor molecules include monosaccharides, oligosaccharides, proteins, lipids, glycoproteins, and glycolipids, respectively.

Sugar chains are known to have an important role in functional expression of biomolecules, and abnormal activities of glycosyltransferases have been shown to be associated with diseases such as cancer, infection, immunological diseases, inflammation, and neurological diseases (Non-Patent Literature 3). Therefore, activity control agents (inhibitors or activators) of glycosyltransferases are expected to be a new therapeutic agent for such diseases.

In order to find activity control agents of glycosyltransferases, it is necessary to measure activities of glycosyltransferases. As methods for measuring activities of glycosyltransferases in the related art, an assay method using sugar molecules labeled with radioactivity or fluorescence and a method in which non-labeled sugar molecules are used to perform separate quantification by instrumental analysis such as liquid chromatography-mass spectrometry (LC-MS) after a reaction have been used

(Non-Patent Literature 4).

However, such methods have problems in that chemical synthesis of labeled sugar donors is necessary, a specific device (a liquid scintillator or LC-MS) is necessary for measurement, it is difficult to perform an assay in a simple and easy manner, and particularly, it is difficult to perform a large number of assays (high-throughput screening) using a microplate.

In addition, in view of costs, such methods are cost-consuming.

On the other hand, a method in which nucleotides produced during glycosyltransferase reactions are quantified by fluorescence polarization using commercially available assay kits (Transcreener (registered trademark) GDP Assay, Transcreener UDP2 Assay, and Transcreener AMP2/GMP2 Assay commercially available from Bellbrook Labs), a method in which absorbance of NADH according to enzymatic coupling reactions is induced and measured (Non-Patent Literatures 5 and 6), and a method in which a phosphate is released and quantification is performed using development of color using malachite green (Non-Patent Literature 7) are known. However, such methods have low detection sensitivity.

In addition, a method in which chemiluminescence of luciferase reaction according to enzymatic coupling is induced for measurement is known (Patent Literature 1), but an operation is complex and sensitivity is unknown.

In addition, a method in which transfer of a sugar labeled with fluorescence is assayed at a high speed using fluorescence polarization has recently been reported (Non-Patent Literature 8). However, this method can be used for only high molecular weight substrates and it is difficult to apply to all glycosyltransferases.

As described above, the methods of the related art have difficulty assaying all glycosyltransferases using a microplate with high sensitivity in a simple and easy manner, and high-throughput screening for activity control agents of glycosyltransferases is difficult.

On the other hand, "kinase" is a general term for enzymes that transfer a terminal phosphate group of nucleoside triphosphate such as ATP (adenosine 5'-triphosphate) to a compound other than water, and catalyze a reaction during

which a phosphate compound is produced, and is a subcategory of phosphotransferases in the group EC2.7 (Non-Patent Literature 9). Exemplary substrate molecules that undergo phosphorylation include sugars, organic acids, lipids, and proteins. Kinases are enzyme molecules that have an important role in signal transduction in vivo. Kinase activities are known to be enhanced in certain types of cancer cells, and inhibitors of kinases are used for cancer treatment as so-called molecularly targeted drugs. In addition, kinases are known to be related to inflammation, immune reactions, diabetes mellitus and the like.

Therefore, kinases are appropriate target molecules for developing therapeutic agents of diseases such as cancer, inflammation, autoimmune diseases, and diabetes mellitus, and research on discovery of new inhibitors is broadly conducted (Non-Patent Literature 10).

In order to find activity control agents (inhibitors or activators) of kinases, it is necessary to measure activities of kinases. As a method for measuring activities of kinases, a method for quantifying phosphorylated products, a method for measuring a decrement of ATP, and a method for quantifying ADP (adenosine 5'-diphosphate) produced from ATP are mainly exemplified.

Exemplary methods for quantifying phosphorylated products include a method in which radioactive products produced from radioactive ATP are measured using radioactivity counting, a method in which phosphorylated products are quantified by thin-layer chromatography (TLC) or high-performance liquid chromatography (HPLC), and a method (for example, QSS Assist (trademark) TR-FRET assay kit commercially available from Carna Biosciences, Inc.) in which phosphorylated products are detected using antibodies and spectroscopically quantified by a time-resolved fluorescence method.

Exemplary methods for measuring a decrement of ATP include a method (for example, "intracelluar" ATP Measuring Reagent (trademark) commercially available form TOYO B-Net Co., Ltd) in which the remaining amount of ATP in a kinase reaction solution is quantified according to chemiluminescence using luciferase.

Exemplary methods for quantifying ADP produced from ATP include 1) a method in which ADP is reconverted into ATP, reacted with luciferase, and quantified by chemiluminescence (for example, ADP-Glo (trademark) Kinase Assay commercially available from Promega Corporation), 2) a method in which anti-ADP antibodies are used for spectroscopical quantification by a time-resolved fluorescence method or fluorescence polarization (for example, Transcreener (registered trademark) ADP TR-FRET Assay, and Transcreener (registered trademark) ADP Assay commercially available from BellBrook Labs), 3) a method in which hydrogen peroxide produced when ADP is reacted with phosphoenolpyruvate, pyruvate kinase, and pyruvate oxidase is further reacted with 10-acetyl-3,7-dihydroxy phenoxazine and peroxidase, and fluorescence is measured (for example, ADP Hunter (trademark) HS Assay commercially available from DiscoveRx), 4) a method in which ADP is reacted with glucose, an ADP-dependent hexokinase, and glucose-6-phosphate dehydrogenase (hereinafter referred to as "G-6-P dehydrogenase") to produce reduced nicotinamide adenine dinucleotide phosphate (NADPH), and absorbance or fluorescence of NADPH is quantified (Patent Literatures 2 and 3), and 5) a method in which fluorescence of resorufin produced when ADP is reacted with glucose, an ADP-dependent hexokinase, G-6-P dehydrogenase, NADP, a diaphorase, and resazurin is quantified (Patent Literature 4).

[Citation List]

[Patent Literature]

[Patent Literature 1]
Japanese Unexamined Patent Application, First Publication No. 2005-46029
[Patent Literature 2]
Japanese Unexamined Patent Application, First Publication No. Hei 9-234098
[Patent Literature 3]
Japanese Unexamined Patent Application, First Publication No. 2011-103825
[Patent Literature 4]
Publication Specification of United States Patent No. 7338775


[Non-Patent Literature]

[Non-Patent Literature 1]
Dictionary of Biochemistry, 4th Edition, p.931, Tokyo Kagaku Dojin, 2007

[Non-Patent Literature 2]
Essentials of Glycobiology, 2nd edition, Part I, Chapter 4, Cold Spring Harbor Laboratory Press, 2009 (http: //www.ncbi.nlm.nih.gov/books/NBK1929/)
[Non-Patent Literature 3]
"Sugar chain engineering can be seen properly," Institute of Advanced Industrial Science and Technology, Hakujitsusha, 2008
[Non-Patent Literature 4]
ChemBioChem, Vol. 11, No. 14, pp. 1939-1949, 2010
[Non-Patent Literature 5]
Anal. Biochem, Vol.102, No.2, pp.441-449, 1980
[Non-Patent Literature 6]
Anal. Biochem, Vol.220, No.1, pp.92-97, 1994
[Non-Patent Literature 7]
Glycobiology, Vol.21, No.6, pp.727-733, 2011
[Non-Patent Literature 8]
Angew. Chem. Int. Ed. Vol.50, No.52, pp.12534-12537, 2011
[Non-Patent Literature 9]
Dictionary of Biochemistry, 4th Edition, p.340, Tokyo Kagaku Dojin, 2007
[Non-Patent Literature 10]
"New challenge for molecularly targeted drug development, drug discovery story of potential molecularly targeted drug and new drug development trend to next-generation drug discovery technology," editors Hideyuki Okano, Takeshi Iwatsubo, Hideyuki Saya, Experimental Medicine, Extra Edition, Vol.27, No.5, Yodosha, 2009

[Summary of Invention]

[Technical Problem]

As described above, various methods are known for measuring activities of kinases. However, since operations are complex, specific devices and instruments are necessary, reagents are expensive, and sensitivity is insufficient, such methods are hard to use for high-throughput screening in which a large number of screening samples are assayed.

In addition, the method 4) in which ADP induces NADPH according to enzymatic coupling using glucose, an ADP-dependent hexokinase, and G-6-P dehydrogenase, and absorbance or fluorescence of NADPH is quantified has problems in that detection sensitivity is low, and it is difficult to perform measurement accurately due to an influence of absorption or autofluorescence of ultraviolet and visible regions near a wavelength of 340 nm derived from a screening sample compound on a measurement value.

In addition, the method 5) in which ADP is reacted with glucose, an ADP-dependent hexokinase, G-6-P dehydrogenase, NADP, a diaphorase, and resazurin, and fluorescence of produced resorufin is measured has problems in that, when a reducing agent such as dithiothreitol (DTT) is included in a reaction solution, since resazurin is reduced to resorufin and background of fluorescence increases, it is difficult to perform measurement accurately.

The reducing agent is added to a reaction system in many cases in order to suppress nonspecific adsorption of a compound or as a stabilizing agent of a reagent such as an enzyme.

In view of the above-described problems, the present invention provides a method for detecting fluorescence or absorbance through which it is possible to measure a fluorescence intensity or absorbance resulting from reduction from resazurin to resorufin with high sensitivity, a method for suppressing background, a method for measuring ADP, a method for measuring activities of ADP-producing enzymes, a method for measuring activities of glycosyltransferases through which it is possible to measure activities of a target enzyme with high sensitivity in a simple and easy manner, an ADP measurement kit, an ADP-producing enzyme activity measurement kit, and a glycosyltransferase activity measurement kit.

[Solution to Problem]

The inventors conducted various studies regarding a method for measuring a fluorescence intensity or absorbance resulting from reduction from resazurin to resorufin with high sensitivity. As a result, it was found that, in the presence of an SH reagent and NADH or NADPH, a fluorescence intensity or absorbance resulting from reduction from resazurin to resorufin using a diaphorase (NAD(P)H dehydrogenase) can be measured with high sensitivity.

In addition, the inventors conducted various studies regarding a method for measuring activities of glycosyltransferases with high sensitivity in a simple and easy manner. As a result, the following was found. When GDP, UDP or CMP produced by a glycosyltransferase is reacted with an NDP kinase (nucleoside 5'-diphosphate kinase) or a CMP

kinase (cytosine 5'-monophosphate kinase) in the presence ofATP, ADP is produced. When the ADP undergoes enzymatic coupling using glucose, an ADP-dependent hexokinase, glucose-6-phosphate dehydrogenase (G-6-P dehydrogenase), a diaphorase, NADP, and resazurin, almost all activities of the glycosyltransferase can be quantified according to fluorescence using a microplate with high sensitivity in a simple, easy and quantitative manner. When a reducing agent such as DTT is included in a reaction solution in a method for quantifying ADP according to fluorescence caused by enzymatic coupling, background of fluorescence development increases, and thus it is difficult to quantify ADP accurately. However, when an SH reagent is included together in the reaction solution during the enzymatic coupling reaction, the background decreases, but there is no influence on the enzymatic coupling reaction itself. Therefore, it is possible to measure ADP with high accuracy, and an ADP fluorescence quantification method in the presence of the SH reagent can be widely used to quantify activities of kinases.

By repeating additional studies based on such findings, a method for detecting fluorescence or absorbance, and a method for measuring activities of glycosyltransferases or ADP-producing enzymes such as kinases with high sensitivity in a simple and easy manner were found, thus completing the present invention.

That is, the present invention provides a method for detecting fluorescence or absorbance, a method for suppressing background, a method for measuring ADP, a method for measuring activities of ADP-producing enzymes, a method for measuring activities of glycosyltransferases, an ADP measurement kit, an ADP-producing enzyme activity measurement kit, and a glycosyltransferase activity measurement kit, which have the following features.

(1) A method for detecting fluorescence or absorbance, including:

reducing, by a diaphorase, resazurin to resorufin, in the presence of an SH reagent and NADH or NADPH, and measuring the resulting fluorescence intensity or absorbance.

(2) The method for detecting fluorescence or absorbance according to item (1),
wherein the SH reagent is a maleimide compound represented by the following General Formula [1] or 2-iodoacetamide.
[Chemical Formula 1]

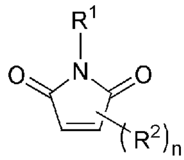

[1]

(In Formula [1],

$R^1$ represents a hydrogen atom, a hydroxyl group, a linear or branched alkyl group that optionally has a substituent group and has 1 to 6 carbon atoms, a linear or branched alkoxy group that optionally has a substituent group and has 1 to 6 carbon atoms, a linear or branched hydroxyalkyl group that optionally has a substituent group and has 1 to 6 carbon atoms, a linear or branched sulfoalkyl group that optionally has a substituent group and has 1 to 6 carbon atoms, or an aryl group that optionally has a substituent group and has 6 to 10 carbon atoms,
$R^2$ represents a hydrogen atom, a hydroxyl group, a halogen atom, a linear or branched alkyl group that optionally has a substituent group and has 1 to 6 carbon atoms, a linear or branched alkoxy group that optionally has a substituent group and has 1 to 6 carbon atoms, a linear or branched hydroxyalkyl group that optionally has a substituent group and has 1 to 6 carbon atoms, or a linear or branched sulfoalkyl group that optionally has a substituent group and has 1 to 6 carbon atoms, and
n represents the number of $R^2$ and is 0 or 1).

(3) A method for suppressing background, including:

an operation in which, when a fluorescence intensity or absorbance caused by reactions of NADH or NADPH, resazurin and a diaphorase in the presence of a reducing agent is measured, the reactions are caused in the presence of an SH reagent.

(4) The method for suppressing background according to item (3), wherein the SH reagent is a maleimide compound represented by the following General Formula [1] or 2-iodoacetamide.

[Chemical Formula 2]

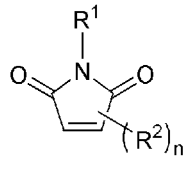

[1]

(In Formula [1], $R^1$, $R^2$, and n have the same meanings as described above).

(5) A method for measuring ADP, including:

a 2-1 process in which glucose is reacted with ADP and an ADP-dependent hexokinase to produce glucose-6-phosphate;
a 2-2 process in which the glucose-6-phosphate obtained in the 2-1 process is reacted with NAD or NADP and glucose-6-phosphate dehydrogenase to produce NADH or NADPH; and
a 2-3 process in which resazurin is reacted with the NADH or NADPH obtained in the 2-2 process and a diaphorase in the presence of an SH reagent, and the resulting fluorescence intensity or absorbance is measured.

(6) The method for measuring ADP according to item (5),
wherein the SH reagent is a maleimide compound represented by the following General Formula [1] or 2-iodoacetamide.

[Chemical Formula 3]

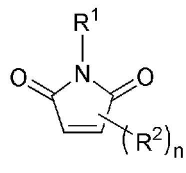

[1]

(In Formula [1], $R^1$, $R^2$, and n have the same meanings as described above).

(7) The method for measuring ADP according to item (5) or (6),
wherein the maleimide compound represented by General Formula [1] is N-ethylmaleimide, maleimide or N-(2-sulfoethyl)maleimide.

(8) A method for measuring activities of ADP-producing enzymes, including:

a 1-1 process in which an ADP-producing enzyme is reacted with a substrate in the presence of ATP to convert the ATP into ADP;
a 2-1 process in which glucose is reacted with the ADP obtained in the 1-1 process and an ADP-dependent hexokinase, to produce glucose-6-phosphate;
a 2-2 process in which the glucose-6-phosphate obtained in the 2-1 process is reacted with NAD or NADP and glucose-6-phosphate dehydrogenase to produce NADH or NADPH; and
a 2-3 process in which resazurin is reacted with the NADH or NADPH obtained in the 2-2 process and a diaphorase in the presence of an SH reagent, and the resulting fluorescence intensity or absorbance is measured.

(9) The method for measuring activities of ADP-producing enzymes according to item (8),
wherein the SH reagent is a maleimide compound represented by the following General Formula [1] or 2-iodoacetamide.

[Chemical Formula 4]

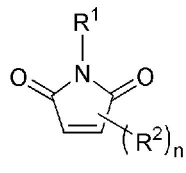

[1]

(In Formula [1], $R^1$, $R^2$, and n have the same meanings as described above).

(10) The method for measuring activities ofADP-producing enzymes according to item (8) or (9),
wherein the maleimide compound represented by General Formula [1] is N-ethylmaleimide, maleimide or N-(2-sulfoethyl)maleimide.

(11) The method for measuring activities ofADP-producing enzymes according to any of items (8) to (10),
wherein the ADP-producing enzyme is at least one type selected from the group including kinases, ATPases, nitrogenases, tetrahydrofolate synthases, acetyl-CoA carboxylase, pyruvate carboxylase, and glutathione synthase.

(12) A method for measuring activities of a glycosyltransferase, including:

a first process in which GDP or UDP produced during a glycosyltransferase reaction is reacted with an NDP kinase in the presence of ATP, or CMP produced during a glycosyltransferase reaction is reacted with NMP kinase or a CMP kinase in the presence of ATP, and thus ADP corresponding to an amount of the GDP, UDP or CMP is produced;
a 2-1 process in which glucose is reacted with the ADP obtained in the first process and an ADP-dependent hexokinase, to produce glucose-6-phosphate;
a 2-2 process in which the glucose-6-phosphate obtained in the 2-1 process is reacted with NAD or NADP and glucose-6-phosphate dehydrogenase to produce NADH or NADPH; and
a 2-3 process in which reactions of the NADH or NADPH obtained in the 2-2 process and a diaphorase are caused in the presence of an SH reagent, and the resulting fluorescence intensity or absorbance is measured.

(13) The method for measuring activities of a glycosyltransferase according to item (12),
wherein the SH reagent is a maleimide compound represented by the following General Formula [1] or 2-iodoacetamide.

[Chemical Formula 5]

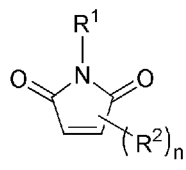

[1]

(In Formula [1], $R^1$, $R^2$, and n have the same meanings as described above)

(14) The method for measuring activities of a glycosyltransferase according to item (12) or (13),
wherein the maleimide compound represented by General Formula [1] is N-ethylmaleimide, maleimide or N-(2-sulfoethyl)maleimide.

(15) The method for measuring activities of a glycosyltransferase according to any of items (12) to (14),
wherein the glycosyltransferase is at least one type selected from the group including fucosyltransferases, mannosyltransferases, glucosyltransferases, galactosyltransferases, glucuronosyltransferases, xylosyltransferases, N-acetylglucosaminyltransferases, N-acetylgalactosaminyltransferases, and sialyltransferases.

(16) An ADP measurement kit including glucose, an ADP-dependent hexokinase, glucose-6-phosphate dehydrogenase, a diaphorase, NAD and/or NADP, resazurin and an SH reagent.

(17) The ADP measurement kit according to item (16),
wherein the SH reagent is a maleimide compound represented by the following General Formula [1] or 2-iodoacetamide.

[Chemical Formula 6]

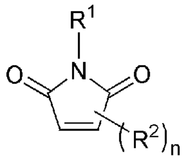
[1]

(In Formula [1], $R^1$, $R^2$, and n have the same meanings as described above).

(18) The ADP measurement kit according to item (16) or (17), wherein the maleimide compound represented by General Formula [1] is N-ethylmaleimide, maleimide or N-(2-sulfoethyl)maleimide.

(19) An ADP-producing enzyme activity measurement kit including glucose, an ADP-dependent hexokinase, glucose-6-phosphate dehydrogenase, a diaphorase, NAD and/or NADP, resazurin and an SH reagent.

(20) The ADP-producing enzyme activity measurement kit according to item (19), wherein the SH reagent is a maleimide compound represented by the following General Formula [1] or 2-iodoacetamide.

[Chemical Formula 7]

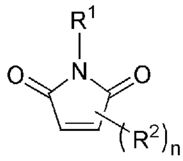
[1]

(In Formula [1], $R^1$, $R^2$, and n have the same meanings as described above).

(21) The ADP-producing enzyme activity measurement kit according to item (20), wherein the maleimide compound represented by General Formula [1] is N-ethylmaleimide, maleimide or N-(2-sulfoethyl)maleimide.

(22) A glycosyltransferase activity measurement kit, including:

a first solution including ATP and NMP kinase, an NDP kinase or a CMP kinase; and

a second solution including glucose, an ADP-dependent hexokinase, glucose-6-phosphate dehydrogenase, a diaphorase, NAD and/or NADP, resazurin, and an SH reagent.

(23) The glycosyltransferase activity measurement kit according to item (22), wherein the first solution further includes a reducing agent.

(24) The glycosyltransferase activity measurement kit according to item (22) or (23), wherein the SH reagent is a maleimide compound represented by the following General Formula [1] or 2-iodoacetamide.

[Chemical Formula 8]

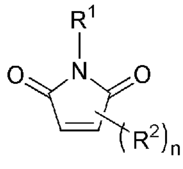
[1]

(In Formula [1], $R^1$, $R^2$, and n have the same meanings as described above).

(25) The glycosyltransferase activity measurement kit according to item (24), wherein the maleimide compound represented by General Formula [1] is N-ethylmaleimide, maleimide or N-(2-sulfoethyl)maleimide.

[Advantageous Effects of Invention]

According to the present invention, it is possible to measure a fluorescence intensity or absorbance resulting from reduction from resazurin to resorufin with high sensitivity.

According to the present invention, it is possible to provide a method for measuring activities of glycosyltransferases or ADP-producing enzymes such as kinases with high sensitivity in a simple and easy manner.

The present invention is suitable for an assay using a microplate. Therefore, according to the present invention, it is possible to perform high-throughput screening for activity control agents of glycosyltransferases or ADP-producing enzymes such as kinases.

[Brief Description of Drawings]

Fig. 1 is a reaction pathway showing a principle of a method of the present invention.
Fig. 2 shows calibration curves of GDP and UDP in an example.
Fig. 3 shows a calibration curve of CMP in an example.
Fig. 4 is a graph showing stability of fluorescence development when GDP, UDP, and CMP are measured in an example.
Fig. 5 shows the results obtained by measuring activities of a fucosyltransferase (human FUT7) in an example.
Fig. 6 shows the results obtained by measuring activities of a galactosyltransferase (human B4GalT1) in an example.
Fig. 7 shows the results obtained by measuring activities of a sialyltransferase (human ST6Gal1) in an example.
Fig. 8 shows the results obtained by measuring inhibitory activities of gallic acid on human FUT7 in an example, in comparison with a case in which HPLC is used for measurement.
Fig. 9 shows a calibration curve of ADP in an example, comparing cases in which N-ethylmaleimide is and is not included during an enzymatic coupling reaction.
Fig. 10 is a graph showing stability of fluorescence development when ADP is quantified in an example.
Fig. 11 shows calibration curves of an ADP solution including 2 mM DTT in an example, comparing cases in which N-ethylmaleimide (NEM) is and is not included in an enzymatic coupling reaction solution.
Fig. 12 shows the results obtained by measuring calibration curves of ADP with and without DTT in an example, comparing cases in which iodoacetamide (IAA) is and is not included in an enzymatic coupling reaction solution.
Fig. 13 shows the results indicating dependence of activities of human CMP kinase 1 (CMPK1) on DTT in an example.
Fig. 14 shows the results obtained by measuring enzyme activities of CMPK1 in the presence of 2 mM DTT in an example, comparing cases in which an enzymatic coupling reaction is caused with and without N-ethylmaleimide for measurement.
Fig. 15 shows the results obtained by measuring kinase activities of human CMPK1 in an example.
Fig. 16 shows the results obtained by measuring inhibitory activities of $Ap_5A$ ($P^1$, $P^5$-Di(adenosine-5')pentaphosphate) on CMPK1 in an example, in comparison with a case in which HPLC is used for measurement.
Fig. 17A shows the results obtained by measuring activities of an ATPase contaminating a commercially available kinase (UMP kinase: UMPK) by HPLC.
Fig. 17B shows the results obtained by measuring activities of an ATPase contaminating a commercially available kinase (UMP kinase: UMPK) in an example, in comparison with the results measured by HPLC.
Fig. 18A shows the results obtained by screening for inhibitors of a galactosyltransferase (human B4GalT1) using a commercially available compound library (LOPAC 1280 (trademark) commercially available from Sigma-Aldrich) in an example. The compound was assayed at 10 μM. Measurement results of a B4GalT1 reaction inhibition rate are shown in Fig. 18A. In the graph, the horizontal axis represents 1280 compounds.
Fig. 18B shows the results obtained by screening for inhibitors of a galactosyltransferase (human B4GalT1) using a commercially available compound library (LOPAC 1280 (trademark) commercially available from Sigma-Aldrich) in an example. The compound was assayed at 10 μM. Measurement results of an inhibitory effect on the assay system itself are shown in Fig. 18B. In the graph, the horizontal axis represents 1280 compounds.
Fig. 18C shows the results obtained by screening for inhibitors of a galactosyltransferase (human B4GalT1) using a commercially available compound library (LOPAC 1280 (trademark) commercially available from Sigma-Aldrich) in an example. The compound was assayed at 10 μM. An inhibition rate considered as a net B4GalT1 inhibitory activity obtained by subtracting a latter inhibition rate from a former inhibition rate is shown in Fig. 18C. In the graph, the horizontal axis represents 1280 compounds.
Fig. 19A shows the results obtained by quantifying ADP in the presence of maleimides and iodoacetamide in an

example.

Fig. 19B shows the results obtained by quantifying ADP in the presence of maleimides and iodoacetamide in an example.

Fig. 19C shows the results obtained by quantifying ADP in the presence of maleimides and iodoacetamide in an example.

Fig. 20A shows the results obtained by quantifying ADP in the presence of a reducing agent DTT, 2-mercaptoethanol, and TCEP in an example.

Fig. 20B shows the results obtained by quantifying ADP in the presence of a reducing agent DTT, 2-mercaptoethanol, and TCEP in an example.

Fig. 20C shows the results obtained by quantifying ADP in the presence of a reducing agent DTT, 2-mercaptoethanol, and TCEP in an example.

[Description of Embodiments]

Exemplary examples of the present invention will be described below, although the present invention is not limited thereto. Additions, omissions, substitutions, and other modifications of the configuration can be made without departing from the scope of the present invention.

<Method for detecting fluorescence or absorbance>

An exemplary form of a method for measuring activities of a glycosyltransferase of the present invention will be described below with reference to Fig. 1. Fig. 1 shows an exemplary reaction pathway according to the method for detecting fluorescence or absorbance of the present invention, and shows a reaction pathway when activities of a glycosyltransferase are measured.

In Fig. 1, as shown in the 2-3 process, in the method for detecting fluorescence or absorbance of the present invention, a diaphorase causes reduction from resazurin to resorufin in the presence of an SH reagent and NADH or NADPH, and the resulting fluorescence intensity or absorbance is measured.

Measurement of a fluorescence intensity or absorbance is performed such that a solution in which, for example, the SH reagent, NADH or NADPH, diaphorase, and resazurin are mixed (hereinafter referred to as a "mixed solution for the 2-3 process") is obtained and undergoes an enzymatic coupling reaction, the NADH or NADPH induces resorufin to develop fluorescence, and fluorescence thereof is quantified. When the mixed solution for the 2-3 process is obtained, the SH reagent, NADH or NADPH, diaphorase, and resazurin used in the enzymatic coupling reaction for measurement can be independently added, or a solution in which some of the components are mixed in advance can be added.

As the diaphorase used in the 2-3 process, any diaphorase originating from animals or plants, originating from microorganisms, or prepared by gene recombination techniques can be used, but a purified diaphorase is preferable.

Concentrations of components after addition are preferably used such as the diaphorase at 0.2 to 20 μg/ml, the NADH or NADPH at 1 to 500 μM or 10 to 500 μM, and the resazurin at 5 to 250 μM. The SH reagent is preferably used at a concentration of in general 1 μM to 100 mM. An amount equivalent to or up to 20 times that of the reducing agent included in the mixed solution for the 2-3 process is suitable.

Exemplary buffer solutions to be used include a tris-hydrochloric acid buffer solution, a Good's buffer such as an HEPES buffer solution and a phosphate buffer solution. The buffer solution having a concentration of 10 to 200 mM and a pH of 7 to 9 is preferable. As an additive, salts such as 10 to 200 mM NaCl, 10 to 200 mM KCl, 5 to 40 mM NaF, 1 to 40 mM $MgCl_2$, 1 to 20 mM $CaCl_2$, and 100 to 500 μM $Na_3$ $VO_4$, surfactants such as 0.01 to 1% TritonX-100, and proteins such as 0.01 to 1% albumin may be added. During the enzymatic coupling reaction of the 2-3 process, a reaction temperature is preferably 20 to 37 °C, and a reaction time is preferably 10 minutes to 3 hours.

When the 2-3 process is performed in the presence of the reducing agent, dithiothreitol (DTT), 2-mercaptoethanol or TCEP is suitable as the reducing agent. The reducing agent is preferably added to be 1μM to 5 mM.

In the mixed solution for the 2-3 process, the SH reagent is added to cause a reaction of the 2-3 process. For example, when the reaction of the 2-3 process is caused while the reducing agent is included in a reaction solution, reduction from resazurin to resorufin is caused by an operation of the reducing agent, and background of fluorescence increases. When the SH reagent is added to the mixed solution for the 2-3 process, it is possible to deactivate the reducing agent, and it is possible to suppress background of fluorescence from increasing.

The SH reagent is a reagent that reacts with a thiol group, and is a compound used for quantification or chemical modification of cysteine residues in proteins. 1) Oxidants such as 5,5'-dithiobis(2-nitrobenzoic acid) (DTNB), 2,2'(4,4')-dipyridyldisulfide, tetrathionate, 2,6-dichlorophenolindophenol (DCIP), and oxidized glutathione, 2) mercaptide-forming agents such as p-mercuribenzoic acid (PMB) and p-mercuribenzene sulfonic acid (PMBS), and 3) alkylating agents such as iodoacetate, iodoacetamide, and N-ethylmaleimide (NEM) are exemplified (refer to Dictionary of Biochemistry, 4th Edition, p. 173, Tokyo Kagaku Dojin, 2007).

As the SH reagent used in the present invention, any reagent that has a reactivity with the reducing agent in the vicinity of a neutral pH range and has no influence on activities of enzymes used in the enzymatic coupling reaction may be used. For example, an $\alpha$-halocarbonyl compound such as iodoacetamide, maleimide derivatives (maleimide compounds) such as N-ethylmaleimide, N-phenylmaleimide, N-(2-chlorophenyl)maleimide, and N-(4-carboxy-3-hydroxyphenyl)maleimide, and allylsulfonate derivatives such as methylvinylsulfone are used. In particular, the $\alpha$-halocarbonyl compound such as iodoacetamide and the maleimide derivatives (the maleimide compounds) such as N-ethylmaleimide are suitable.

As the SH reagent used in the method for detecting fluorescence or absorbance of the present invention, a maleimide compound represented by the following General Formula [1] or 2-iodoacetamide is preferable.

[Chemical Formula 9]

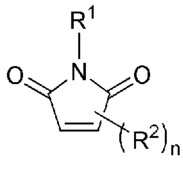
[1]

(In Formula [1],

$R^1$ represents a hydrogen atom, a hydroxyl group, a linear or branched alkyl group that optionally has a substituent group and has 1 to 6 carbon atoms, a linear or branched alkoxy group that optionally has a substituent group and has 1 to 6 carbon atoms, a linear or branched hydroxyalkyl group that optionally has a substituent group and has 1 to 6 carbon atoms, a linear or branched sulfoalkyl group that optionally has a substituent group and has 1 to 6 carbon atoms, or an aryl group that optionally has a substituent group and has 6 to 10 carbon atoms,

$R^2$ represents a hydrogen atom, a hydroxyl group, a halogen atom, a linear or branched alkyl group that optionally has a substituent group and has 1 to 6 carbon atoms, a linear or branched alkoxy group that optionally has a substituent group and has 1 to 6 carbon atoms, a linear or branched hydroxyalkyl group that optionally has a substituent group and has 1 to 6 carbon atoms, or a linear or branched sulfoalkyl group that optionally has a substituent group and has 1 to 6 carbon atoms, and

n represents the number of $R^2$ and is 0 or 1).

As the linear or branched alkyl group having 1 to 6 carbon atoms of $R^1$ and $R^2$, a methyl group, an ethyl group, an n-propyl group, an isopropyl group, an n-butyl group, an isobutyl group, a sec-butyl group, a tert-butyl group, a pentyl group, an isopentyl group, a neopentyl group, an n-hexyl group, and an isohexyl group are exemplified.

As the linear or branched alkoxy group having 1 to 6 carbon atoms of $R^1$ and $R^2$, a methoxy group, an ethoxy group, an n-propoxy group, an iso-propoxy group, an n-butoxy group, a tert-butoxy group, and an n-hexyloxy group are exemplified.

The linear or branched hydroxyalkyl group having 1 to 6 carbon atoms of $R^1$ and $R^2$ is an alkyl group in which at least one hydrogen atom is substituted with an independently selected hydroxyl group. As the alkyl group of the linear or branched hydroxyalkyl group having 1 to 6 carbon atoms of $R^1$ and $R^2$. the same as those exemplified in the linear or branched alkyl group having 1 to 6 carbon atoms of $R^1$ and $R^2$ may be used.

The linear or branched sulfoalkyl group having 1 to 6 carbon atoms of $R^1$ and $R^2$ is an alkyl group in which at least one hydrogen atom is substituted with an independently selected sulfo group. As the alkyl group of the linear or branched sulfoalkyl group having 1 to 6 carbon atoms of $R^1$ and $R^2$, the same as those exemplified in the linear or branched alkyl group having 1 to 6 carbon atoms of $R^1$ and $R^2$ may be used.

As the aryl group having 6 to 10 carbon atoms of $R^1$, a phenyl group, a naphthyl group and the like may be exemplified.

The halogen atom of $R^2$ is an element belonging to Group 17 in the periodic table, for example, F, Cl, Br, and I.

As the linear or branched alkyl group having 1 to 6 carbon atoms of $R^1$ and $R^2$, an alkyl group having 1 to 4 carbon atoms is preferable, and an alkyl group having 1 to 3 carbon atoms is more preferable.

As the linear or branched alkoxy group having 1 to 6 carbon atoms of $R^1$ and $R^2$, an alkoxy group having 1 to 4 carbon atoms is preferable, and an alkoxy group having 1 to 3 carbon atoms is more preferable.

As the linear or branched hydroxyalkyl group having 1 to 6 carbon atoms of $R^1$ and $R^2$, a hydroxyalkyl group having 1 to 4 carbon atoms is preferable, and a hydroxyalkyl group having 1 to 3 carbon atoms is more preferable.

As the linear or branched sulfoalkyl group having 1 to 6 carbon atoms of $R^1$ and $R^2$, a sulfoalkyl group having 1 to 4 carbon atoms is preferable, and a sulfoalkyl group having 1 to 3 carbon atoms is more preferable.

The substituent group that may be included in the alkyl group, the alkoxy group, the hydroxyalkyl group, or the

sulfoalkyl group of $R^1$ and $R^2$ replaces a hydrogen atom (H) in a hydrocarbon group. As the substituent group, a halogen atom, a hydroxyl group, and an amino group are exemplified.

The substituent group that may be included in the aryl group of $R^1$ replaces a hydrogen atom (H) in the aryl group. As the substituent group, a linear or branched alkyl group having 1 to 6 carbon atoms, a carboxyl group, a halogen atom, a hydroxyl group, an amino group, and a nitro group are exemplified.

<Method for suppressing background>

An exemplary form of a method for suppressing background of the present invention will be described below with reference to Fig. 1. Fig. 1 is an exemplary reaction pathway according to the method for suppressing background of the present invention, and a reaction pathway when activities of a glycosyltransferase are measured.

In the method for suppressing background of the present invention, when reactions ofNADH or NADPH, resazurin and a diaphorase are caused in the presence of the reducing agent as shown in the 2-3 process in Fig. 1 and a fluorescence intensity or absorbance caused by the reaction is measured, the reaction is caused in the presence of the SH reagent.

When the reaction of the 2-3 process is caused while the reducing agent is included, reduction from resazurin to resorufin is caused by an operation of the reducing agent, and background of fluorescence increases. When the SH reagent is added to the mixed solution for the 2-3 process, it is possible to deactivate the reducing agent, and it is possible to suppress background of fluorescence from increasing.

As a reaction system of a target whose background is suppressed, a mixed solution in which the reducing agent is added to a solution in which, for example, an SH reagent, NADH or NADPH, a diaphorase, and resazurin are mixed (hereinafter referred to as a "mixed solution for the 2-3 process") is exemplified. When the mixed solution in which the reducing agent is added to the mixed solution for the 2-3 process is obtained, the SH reagent, NADH or NADPH, diaphorase, and resazurin used in the enzymatic coupling reaction for measurement can be independently added, or a solution in which some of the components are mixed in advance can be added.

In the mixed solution, when the reaction shown in the 2-3 process is caused to proceed, NADH or NADPH induces resorufin to develop fluorescence. Here, since the SH reagent is added to the mixed solution, it is possible to suppress a background signal other than a signal to be detected from increasing when the fluorescence development is quantified.

As the diaphorase used in the 2-3 process, any diaphorase originating from animals or plants, originating from microorganisms, or prepared by gene recombination techniques can be used, but a purified diaphorase is preferable.

Concentrations of components after addition are preferably used such as the reducing agent at 1 $\mu$M to 5 mM, the diaphorase at 0.2 to 20 $\mu$g/ml, the NADH or NADPH at 1 to 500 $\mu$M or 10 to 500 $\mu$M, and the resazurin at 5 to 250 $\mu$M. The SH reagent is preferably used at a concentration of in general 1 $\mu$M to 100 mM. An amount equivalent to or up to 20 times that of the reducing agent included in the mixed solution for the 2-3 process is suitable.

Exemplary buffer solutions to be used include a tris-hydrochloric acid buffer solution, a Good's buffer such as an HEPES buffer solution, and a phosphate buffer solution. The buffer solution having a concentration of 10 to 200 mM and a pH of 7 to 9 is preferable. As an additive, salts such as 10 to 200 mM NaCl, 10 to 200 mM KCl, 5 to 40 mM NaF, 1 to 40 mM $MgCl_2$, 1 to 20 mM $CaCl_2$, and 100 to 500 $\mu$M $Na_3VO_4$, surfactants such as 0.01 to 1% TritonX-100, and proteins such as 0.01 to 1% albumin may be added. During the enzymatic coupling reaction of the 2-3 process, a reaction temperature is preferably 20 to 37 °C, and a reaction time is preferably 10 minutes to 3 hours.

When the 2-3 process is performed in the presence of the reducing agent, dithiothreitol (DTT), 2-mercaptoethanol or TCEP is suitable as the reducing agent. The reducing agent is preferably added to be 1 $\mu$M to 5 mM.

As the SH reagent used in the method for suppressing background of the present invention, any reagent that has a reactivity with the reducing agent in the vicinity of a neutral pH range and has no influence on activities of enzymes used in the enzymatic coupling reaction may be used. For example, an $\alpha$-halocarbonyl compound such as iodoacetamide, maleimide derivatives such as N-ethylmaleimide, and allylsulfonate derivatives such as methylvinylsulfone are used. In particular, an $\alpha$-halocarbonyl compound such as iodoacetamide and maleimide derivatives such as N-ethylmaleimide are suitable.

As the SH reagent used in the present invention, the maleimide compound represented by General Formula [1] or 2-iodoacetamide is preferable.

<Method for measuring ADP>

An exemplary form of a method for measuring ADP of the present invention will be described below with reference to Fig. 1. Fig. 1 is an exemplary reaction pathway according to the method for measuring ADP of the present invention and is a reaction pathway when ADP produced during an enzymatic reaction of glycosyltransferase is measured.

In Fig. 1, as shown in a second process including a 2-1 process, a 2-2 process, and a 2-3 process, the method

for measuring ADP of the present invention includes the 2-1 process in which glucose is reacted with ADP and an ADP-dependent hexokinase to produce glucose-6-phosphate, the 2-2 process in which the glucose-6-phosphate obtained in the 2-1 process is reacted with NAD or NADP and glucose-6-phosphate dehydrogenase to produce NADH or NADPH, and the 2-3 process in which resazurin is reacted with the NADH or NADPH obtained in the 2-2 process and a diaphorase in the presence of the SH reagent, and the resulting fluorescence intensity or absorbance is measured.

Measurement of ADP is performed such that, for example, a mixed solution in which ADP is added to a solution in which the glucose, ADP-dependent hexokinase, G-6-P dehydrogenase, diaphorase, NAD or NADP, and resazurin used in the enzymatic coupling reaction for quantifying ADP are mixed (hereinafter referred to as a "mixed solution for the second process") is obtained and undergoes an enzymatic coupling reaction, ADP induces resorufin to develop fluorescence, and fluorescence thereof is quantified. When the mixed solution for the second process is obtained, the glucose, ADP-dependent hexokinase, G-6-P dehydrogenase, diaphorase, NAD or NADP, and resazurin used in the enzymatic coupling reaction for measurement can be independently added, or a solution in which some of the components are mixed in advance can be added.

Concentrations of components after addition are preferably used such as the glucose at 0.1 to 10 mM, the ADP-dependent hexokinase at 5 to 500 μg/ml, the G-6-P dehydrogenase at 1 to 100 μg/ml, the diaphorase at 0.2 to 20 μg/ml, the NAD or NADP at 1 to 500 μM or 10 to 500 μM, and the resazurin at 5 to 250 μM. The SH reagent is preferably used at a concentration of in general 1 μM to 100 mM. An amount equivalent to or up to 20 times that of the reducing agent included in the mixed solution for the 2-3 process is suitable.

Exemplary buffer solutions to be used include a tris-hydrochloric acid buffer solution, a Good's buffer such as an HEPES buffer solution, and a phosphate buffer solution. The buffer solution having a concentration of 10 to 200 mM and a pH of 7 to 9 is preferable. As an additive, salts such as 10 to 200 mM NaCl, 10 to 200 mM KCl, 5 to 40 mM NaF, 1 to 40 mM $MgCl_2$, 1 to 20 mM $CaCl_2$, and 100 to 500 μM $Na_3VO_4$, surfactants such as 0.01 to 1% TritonX-100, and proteins such as 0.01 to 1% albumin may be added. During the enzymatic coupling reaction of the second process, a reaction temperature is preferably 20 to 37 °C and a reaction time is preferably 10 minutes to 3 hours.

When the 2-3 process is performed in the presence of the reducing agent, dithiothreitol (DTT), 2-mercaptoethanol or TCEP is suitable as the reducing agent. The reducing agent is preferably added to be 1 μM to 5 mM

In addition, as the ADP-dependent hexokinase, G-6-P dehydrogenase, and diaphorase used in the second process, any ADP-dependent hexokinase, G-6-P dehydrogenase, and diaphorase originating from animals or plants, originating from microorganisms, or prepared by gene recombination techniques can be used, but a purified ADP-dependent hexokinase, G-6-P dehydrogenase, and diaphorase are preferable.

In the method for measuring ADP of the present invention, the SH reagent is added to the mixed solution for the second process to cause the reaction of the second process. For example, when the enzymatic coupling reaction of the second process is caused while the reducing agent is included, reduction from resazurin to resorufin is caused by an operation of the reducing agent, and background of fluorescence increases. When the SH reagent is added to the mixed solution for enzymatic coupling of the second process, it is possible to deactivate the reducing agent, and it is possible to suppress background of fluorescence from increasing.

When the method for measuring ADP of the present invention is performed in the presence of the reducing agent, dithiothreitol (DTT), 2-mercaptoethanol or TCEP is suitable as the reducing agent. The reducing agent is preferably added to be 1 μM to 5 mM.

As the SH reagent used in the method for measuring ADP of the present invention, any reagent that has a reactivity with the reducing agent in the vicinity of a neutral pH range and has no influence on activities of enzymes used in the enzymatic coupling reaction may be used. For example, an α-halocarbonyl compound such as iodoacetamide, maleimide derivatives such as N-ethylmaleimide, and allylsulfonate derivatives such as methylvinylsulfone are used. In particular, an α-halocarbonyl compound such as iodoacetamide and maleimide derivatives such as N-ethylmaleimide are suitable.

As the SH reagent used in the present invention, the maleimide compound represented by General Formula [1] or 2-iodoacetamide is preferable.

The method for measuring ADP of the present invention can be used as a method for measuring ADP and measuring broad activities of enzymes that produce ADP during a reaction.

<Method for measuring activities of ADP-producing enzymes>

On the other hand, the method for quantifying ADP of the second process of the present invention can be used as a method for measuring broad activities of enzymes that produce ADP during a reaction such as kinases.

That is, in the method for measuring activities of ADP-producing enzymes of the present invention, ADP produced during an ADP-producing enzymatic reaction is measured according to fluorescence of resorufin produced when reactions of glucose, an ADP-dependent hexokinase, glucose-6-phosphate delrydrogenase, a diaphorase, NADP, and resazurin are caused in the presence of the SH reagent.

The method for measuring activities of ADP-producing enzymes of the present invention includes a 1-1 process and the second process including the 2-1 process, the 2-2 process and the 2-3 process shown in Fig. 1.

The method includes the 1-1 process in which an ADP-producing enzyme is reacted with a substrate in the presence of ATP to convert ATP into ADP, the 2-1 process in which glucose is reacted with the ADP obtained in the 1-1 process and an ADP-dependent hexokinase to produce glucose-6-phosphate, the 2-2 process in which the glucose-6-phosphate obtained in the 2-1 process is reacted with NAD or NADP and glucose-6-phosphate dehydrogenase to produce NADH or NADPH, and the 2-3 process in which resazurin is reacted with the NADH or NADPH obtained in the 2-2 process and a diaphorase in the presence of the SH reagent, and the resulting fluorescence intensity or absorbance is measured.

Exemplary examples of the present invention will be described below using a kinase as an example.

As a kinase to be measured, any kinase that is extracted from animal and plant tissues and cells, originating from microorganisms, or prepared by gene recombination techniques can be used, but a purified kinase is preferable.

Exemplary kinases include protein kinase A, protein kinase C, calmodulin kinase, and casein kinase.

In the present invention, a kinase reaction during which enzyme activities are to be measured is caused by mixing a kinase, phosphate donor molecules (generally ATP) and phosphate acceptor molecules (substrate molecules corresponding to the kinase) in a buffer solution. As respective addition concentrations, the kinase is preferably added at a concentration at which an enzymatic reaction rate becomes about 1 to 50%, the phosphate donor molecules (mainly ATP) are preferably added at 10 to 1000 μM, and the phosphate acceptor molecules are preferably added at 5 to 500 μM. Exemplary buffer solutions used in the kinase reaction include a tris-hydrochloric acid buffer solution, a Good's buffer such as an HEPES buffer solution and a phosphate buffer solution. In the buffer solution, a concentration is preferably 10 to 200 mM, and a pH is preferably 7 to 9. As an additive, as necessary, salts such as 10 to 200 mM NaCl, 10 to 200 mM KCl, 5 to 40 mM NaF, 1 to 20 mM $MgCl_2$, 1 to 20 mM $MnCl_2$, 1 to 20 mM $CaCl_2$, and 100 to 500 μM $Na_3VO_4$, surfactants such as 0.01 to 1% TritonX-100, and proteins such as 0.01 to 1% albumin may be added.

In addition, it is preferable that the reducing agent such as DTT, 2-mercaptoethanol, or TCEP (Tris(2-carboxyethyl)phosphine hydrochloride) be added to cause a reaction when the kinase to be measured has reducing agent requirements, or as an activator (an activating agent) for increasing (activating and accelerating) enzyme activities, or as a stabilizing agent of a reagent such as an enzyme in order to suppress nonspecific adsorption of a compound during screening. Addition concentrations of the DTT, 2-mercaptoethanol or TCEP depend on the reducing agent requirements of the kinase to be measured, but a range of 0.1 to 10 mM is preferable. A reaction temperature is preferably 20 to 37 °C, and a reaction time is preferably 10 minutes to 3 hours. In the reaction solution after the kinase reaction, the ADP preferably has a concentration in a range of 0.1 to 50 μM, and a range of 0.5 to 25 μM is more preferable. When the reaction solution has a concentration higher than this range, it is preferable that the solution be diluted to be in the range.

The ADP produced during the kinase reaction is quantified according to fluorescence of resorufin produced when the glucose, ADP-dependent hexokinase, G-6-P dehydrogenase, diaphorase, NADP, and resazurin are added to cause enzymatic coupling. Such components can be separately added, or can be mixed in advance (hereinafter referred to as a "mixed solution for enzymatic coupling," having the same composition as the mixed solution for the second process described above) and then added to the reaction solution after the kinase reaction at once.

As final concentrations of such components when added to the reaction solution after the kinase reaction, the glucose at 0.5 to 5 mM, the ADP-dependent hexokinase at 5 to 100 μg/ml, the G-6-P dehydrogenase at 0.5 to 10 μg/ml, the diaphorase at 0.2 to 20 μg/ml or 0.5 to 10 μg/ml, the NADP at 20 to 400 μM, the resazurin at 5 to 250 μM or 10 to 200 μM are preferably prepared. The SH reagent is preferably used at a concentration of in general 1 μM to 100 mM. An amount equivalent to or up to 20 times that of the reducing agent included in the mixed solution for enzymatic coupling is suitable.

Exemplary buffer solutions used in addition include a tris-hydrochloric acid buffer solution, a Good's buffer such as an HEPES buffer solution, and a phosphate buffer solution. The buffer solution having a concentration of 10 to 200 mM and a pH of 7 to 9 is preferable. As an additive, salts such as 10 to 200 mM NaCl, 10 to 200 mM KCl, 5 to 40 mM NaF, 1 to 40 mM $MgCl_2$, 1 to 20 mM $MnCl_2$, 1 to 20 mM $CaCl_2$, and 100 to 500 μM $Na_3VO_4$, surfactants such as 0.01 to 1% TritonX-100, and proteins such as 0.01 to 1% albumin may be added. During the enzymatic coupling reaction, a reaction temperature is preferably 20 to 37 °C, and a reaction time is preferably 10 minutes to 3 hours.

When the 2-3 process is performed in the presence of the reducing agent, dithiothreitol (DTT), 2-mercaptoethanol or TCEP is suitable as the reducing agent. The reducing agent is preferably added to be 1 μM to 5 mM

In order to exhibit enzyme activities with many types of kinases, it is necessary to add the reducing agent. However, when an enzymatic coupling reaction for quantifying ADP is caused while the reducing agent is included, reduction from resazurin to resorufin is caused by an operation of the reducing agent, and background of fluorescence increases.

In the present invention, when the SH reagent described above is added to the mixed solution for enzymatic coupling, it is possible to deactivate the reducing agent and suppress background from increasing for measurement. As the SH reagent used for measuring kinase activities, any reagent that has a reactivity with the reducing agent in the

vicinity of a neutral pH range and has no influence on activities of enzymes used in the enzymatic coupling reaction may be used. For example, an $\alpha$-halocarbonyl compound such as iodoacetamide or maleimide derivatives such as N-ethylmaleimide are used. As an amount to be added for the enzymatic coupling reaction, an amount equivalent to or up to 20 times that of the reducing agent used in the kinase reaction is suitable.

As the SH reagent used in the present invention, the maleimide compound represented by General Formula [1] or 2-iodoacetamide is preferable.

A method for quantifying ADP of the present invention can be used to measure activities of ADP-producing enzymes other than kinases. As the ADP-producing enzymes other than kinases, ATPases, nitrogenases, tetrahydrofolate synthases, acetyl-CoA carboxylase, pyruvate carboxylase, and glutathione synthase are exemplified.

A container used for measuring activities of glycosyltransferases or ADP-producing enzymes such as kinases is not limited as long as a reaction is caused therein, but a microplate is suitable. Any type among 96-well, 384-well, and 1536-well microplates is used.

First, the microplate is used to cause a reaction of a glycosyltransferase or an ADP-producing enzyme such as a kinase. In the case of a glycosyltransferase, a mixed solution for a first process is added to the reaction solution to cause a reaction of the first process. Next, the mixed solution for a second process is added to cause a reaction of the second process. Then, fluorescence of products (resorufin) is measured using a microplate reader.

In the case of a kinase, the mixed solution for enzymatic coupling is added to a kinase reaction solution to cause a reaction. Then, fluorescence of resorufin is similarly measured using the microplate reader. When fluorescence of the resorufin is measured, an excitation wavelength is preferably 530 to 570 nm, and a fluorescence wavelength is preferably 580 to 610 nm.

A reaction during which ADP is caused to develop fluorescence by the method of the present invention is almost completed within 30 minutes when a reaction temperature is 25 °C. Since a fluorescence value is stable for about 2 hours thereafter, an operation of suspending the reaction is not particularly necessary when fluorescence is measured.

<Method for measuring activities of a glycosyltransferase>

An exemplary form of a method for measuring activities of a glycosyltransferase of the present invention will be described below with reference to Fig. 1. Fig. 1 is a reaction pathway showing a principle of a method for measuring activities of a glycosyltransferase of the present invention.

As shown in Fig. 1, the method for measuring activities of a glycosyltransferase of the present invention includes a first process in which GDP or UDP produced during a glycosyltransferase reaction is reacted with NDP kinase in the presence of ATP, or CMP produced during a glycosyltransferase reaction is reacted with a CMP kinase in the presence of ATP, and thus ADP corresponding to an amount of GDP, UDP or CMP is produced, and a second process in which the ADP is quantified according to fluorescence caused by the enzymatic coupling reaction using glucose, an ADP-dependent hexokinase, glucose-6-phosphate dehydrogenase, a diaphorase, NADP, and resazurin.

That is, the method for measuring activities of a glycosyltransferase of the present invention includes the first process in which the GDP or UDP produced during the glycosyltransferase reaction is reacted with the NDP kinase in the presence of the ATP, or the CMP produced during the glycosyltransferase reaction is reacted with the NMP kinase or CMP kinase in the presence of the ATP, and thus the ADP corresponding to the amount of GDP, UDP or CMP is produced, a 2-1 process in which glucose is reacted with the ADP obtained in the first process and an ADP-dependent hexokinase to produce glucose-6-phosphate, a 2-2 process in which the glucose-6-phosphate obtained in the 2-1 process is reacted with NAD or NADP and glucose-6-phosphate dehydrogenase to produce NADH or NADPH, and a 2-3 process in which reactions of the NADH or NADPH obtained in the 2-2 process and a diaphorase are caused in the presence of the SH reagent, and the resulting fluorescence intensity or absorbance is measured.

As the glycosyltransferase to be measured, any glycosyltransferase that is derived from animals or plants, derived from microorganisms, derived in vivo, or prepared by gene recombination techniques can be used, but a purified glycosyltransferase is preferable.

As the glycosyltransferase to be measured, at least one type selected from among the group including fucosyltransferases, mannosyltransferases, glucosyltransferases, galactosyltransferases, glucuronosyltransferases, xylosyltransferases, N-acetylglucosaminyltransferases,
N-acetylgalactosaminyltransferases, and sialyltransferases is preferable.

In addition, as the NDP kinase, CMP kinase, ADP-dependent hexokinase, G-6-P dehydrogenase, and diaphorase used in the first process and the second process, any NDP kinase, CMP kinase, ADP-dependent hexokinase, G-6-P dehydrogenase, and diaphorase originating from animals or plants, originating from microorganisms, or prepared by gene recombination techniques can be used, but a purified NDP kinase, CMP kinase, ADP-dependent hexokinase, G-6-P dehydrogenase, and diaphorase are preferable.

When a sugar (for example, fucose, mannose, glucose, galactose, glucuronic acid, xylose, N-acetylglucosamine, N-acetylgalactosamine, and sialic acid) is transferred from sugar donor molecules such as GDP-fucose, GDP-mannose, UDP-glucose, UDP-galactose, UDP-glucuronic acid, UDP-xylose, UDP-N-acetylglucosamine, UDP-N-acetylgalactosamine, and CMP-sialic acid to sugar receptor molecules according to an action of the glycosyltransferase, nucleotides (GDP, UDP or CMP) are released from the sugar donor molecules.

The present invention is provided to measure activities of a glycosyltransferase by quantifying GDP, UDP or CMP according to fluorescence using the enzymatic coupling reaction.

In the related art, it was difficult to quantify GDP, UDP or CMP with high sensitivity in a simple and easy manner. In the present invention, it is possible to measure GDP, UDP or CMP with high sensitivity in a simple and easy manner according to the enzymatic coupling reaction including the following two processes.

First process: when nucleotides produced from the sugar donor molecules during transglucosylation are GDP or UDP, the NDP kinase is reacted in the reaction solution after transglucosylation in the presence of the ATP, the GDP and UDP are converted into GTP and UTP, respectively, and ADP is quantitatively produced. When nucleotides produced from the sugar donor molecules during transglucosylation are CMP, the CMP kinase is reacted in the reaction solution after transglucosylation in the presence of the ATP, the CMP is converted into CDP, and ADP is quantitatively produced.

Second process: the ADP produced in the first process induces fluorescent resorufin to develop fluorescence according to the enzymatic coupling reaction using the glucose, ADP-dependent hexokinase, G-6-P dehydrogenase, diaphorase, NADP or NAD, and resazurin and is quantified according to fluorescence.

In the present invention, a transglucosylation reaction during which enzyme activities are to be measured is caused by mixing the glycosyltransferase, sugar donor molecules, and sugar receptor molecules in a buffer solution. The glycosyltransferase is preferably added at a concentration at which an enzymatic reaction rate becomes about 1 to 50%, the sugar donor molecules are preferably added at 10 to 500 μM, and the sugar receptor molecules are preferably added at 10 to 5000 μM.

Exemplary buffer solutions used in transglucosylation include a tris-hydrochloric acid buffer solution, a Good's buffer such as an HEPES buffer solution and a phosphate buffer solution. The buffer solution having a concentration of 10 to 200 mM and a pH of 6 to 9 is preferable. As an additive, salts such as 10 to 200 mM NaCl, 10 to 200 mM KCl, 5 to 40 mM NaF, 1 to 40 mM $MgCl_2$, 1 to 20 mM $MnCl_2$, 1 to 20 mM $CaCl_2$, and 100 to 500 μM $Na_3 VO_4$, surfactants such as 0.01 to 1% TritonX-100, and proteins such as 0.01 to 1% albumin may be added.

A reaction temperature is preferably 20 to 37 °C, and a reaction time is preferably 10 minutes to 3 hours. Free nucleotides in the reaction solution after transglucosylation have a concentration that is preferably in a range of 0.1 to 100 μM and more preferably in a range of 0.5 to 50 μM. When the reaction solution has a concentration higher than 100 μM, it is preferable that the solution be diluted to be in that range.

As a transglucosylation result, in order to quantify the nucleotides (GDP, UDP or CMP) released from the sugar donor molecules, in the first process, the ATP, and NDP kinase or CMP kinase are added to the reaction solution after transglucosylation, and a phosphate exchange reaction is caused to produce the ADP.

An NDP kinase is an enzyme that causes phosphorylation to convert GDP and UDP into GTP and UTP, respectively, in the presence of ATP, and produces ADP having the same molar quantity as the GTP or UTP.

A CMP kinase is an enzyme that causes phosphorylation to convert CMP into CDP in the presence of ATP, and produces ADP having the same molar quantity as the CDP. In the present invention, any enzyme that causes phosphorylation to convert CMP into CDP in the presence ofATP and produces ADP having the same molar quantity as the CDP, the enzyme can be used as the CMP kinase. For example, since an enzyme called NMP kinase (nucleoside 5'-monophosphate kinase) also has this enzyme activity, it can be used as the CMP kinase.

The ATP and NDP kinase or CMP kinase added in the first process may be independently added, or added as a mixed solution. In any case, ATP is preferably added at a molar concentration twice that of free nucleotides included in a transglucosylation solution or more and a concentration less than about 1 mM.

The NDP kinases or CMP kinase is preferably added at 0.2 to 20 μg/ml. Exemplary buffer solutions used for such addition include a tris-hydrochloric acid buffer solution, a Good's buffer such as an HEPES buffer solution and a phosphate buffer solution. The buffer solution having a concentration of 10 to 200 mM and a pH of 7 to 9 is preferable. As an additive, salts such as 10 to 200 mM NaCl, 10 to 200 mM KCl, 5 to 40 mM NaF, 1 to 40 mM $MgCl_2$, 1 to 20 mM $CaCl_2$, and 100 to 500 μM $Na_3VO_4$, surfactants such as 0.01 to 1% TritonX-100, and proteins such as 0.01 to 1% albumin may be added.

A reaction temperature is preferably 20 to 37 °C, and a reaction time is preferably 10 minutes to 3 hours. Note that, since enzyme activities of the CMP kinase increase according to addition of the reducing agent, it is preferable that the reducing agent be added at the same time, and ADP be produced in the presence of the reducing agent when the CMP kinase is used in the first process. Note that, in addition to a case in which the kinase to be measured has reducing agent requirements, it is preferable that the reducing agent be added to cause a reaction as an activator (an activating agent) for increasing (activating and accelerating) enzyme activities, or as a stabilizing agent of a reagent such as an enzyme in order to suppress nonspecific adsorption of a compound during screening. Dithiothreitol (DTT), 2-mercaptoethanol or TCEP is suitable as the reducing agent. An amount added is preferably 1 μM to 5 mM, and more preferably 0.1 to 5 mM.

In the second process, when the glucose, ADP-dependent hexokinase, G-6-P dehydrogenase, diaphorase, NADP and resazurin are added to the reaction solution obtained in the first process to cause the enzymatic coupling reaction, the ADP induces the resorufin to develop fluorescence, and this fluorescence is quantified. The glucose, ADP-dependent hexokinase, G-6-P dehydrogenase, diaphorase, NADP or NAD, and resazurin used in the enzymatic coupling reaction for quantifying the ADP can be independently added, and a solution in which components are mixed in advance (hereinafter referred to as a "mixed solution for the second process") can be added at once.

Concentrations of the components after addition are preferably used such as the glucose at 0.1 to 10 mM, the ADP-dependent hexokinase at 5 to 500 μg/ml, the G-6-P dehydrogenase at 1 to 100 μg/ml, the diaphorase at 0.2 to 20 μg/ml, the NADP at 10 to 500 μM, and the resazurin at 5 to 250 μM. The SH reagent is preferably used at a concentration of in general 1 μM to 100 mM. An amount equivalent to or up to 20 times that of the reducing agent included in the mixed solution for enzymatic coupling is suitable.

Exemplary buffer solutions to be used include a tris-hydrochloric acid buffer solution, a Good's buffer such as an HEPES buffer solution, and a phosphate buffer solution. The buffer solution having a concentration of 10 to 200 mM and a pH of 7 to 9 is preferable. As an additive, salts such as 10 to 200 mM NaCl, 10 to 200 mM KCl, 5 to 40 mM NaF, 1 to 40 mM $MgCl_2$, 1 to 20 mM $CaCl_2$, and 100 to 500 μM $Na_3VO_4$, surfactants such as 0.01 to 1% TritonX-100, and proteins such as 0.01 to 1% albumin may be added. During the enzymatic coupling reaction of the second process, a reaction temperature is preferably 20 to 37 °C, and a reaction time is preferably 10 minutes to 3 hours.

When the 2-3 process is performed in the presence of the reducing agent, dithiothreitol (DTT), 2-mercaptoethanol or TCEP is suitable as the reducing agent. The reducing agent is preferably added to be 1 μM to 5 mM.

In addition, when the reducing agent is added in the first process, it is preferable that the SH reagent be added to the mixed solution for the second process to cause the reaction of the second process. When the enzymatic coupling reaction of the second process is caused while the reducing agent is included, reduction from resazurin to resorufin is caused by an operation of the reducing agent, and background of fluorescence increases. When the SH reagent is added to the mixed solution for enzymatic coupling of the second process, it is possible to deactivate the reducing agent, and it is possible to suppress background of fluorescence from increasing.

The SH reagent is a reagent that reacts with a thiol group, and is a compound used for quantification or chemical modification of cysteine residues in proteins. 1) Oxidants such as 5,5'-dithiobis (2-nitrobenzoic acid) (DTNB), 2,2'(4,4')-dipyridyldisulfide, tetrathionate, 2,6-dichlorophenolindophenol (DCIP), and oxidized glutathione, 2) mercaptide-forming agents such as p-mercuribenzoic acid (PMB) and p-mercuribenzene sulfonic acid (PMBS), and 3) alkylating agents such as iodoacetate, iodoacetamide, and N-ethylmaleimide (NEM) are exemplified (refer to Dictionary of Biochemistry, 4th Edition, p. 173, Tokyo Kagaku Dojin, 2007).

As the SH reagent used in the present invention, any reagent that has a reactivity with the reducing agent in the vicinity of a neutral pH range and has no influence on activities of enzymes used in the enzymatic coupling reaction may be used. For example, an α-halocarbonyl compound such as iodoacetamide, maleimide derivatives such as N-ethylmaleimide, and allylsulfonate derivatives such as methylvinylsulfone are used. In particular, an α-halocarbonyl compound such as iodoacetamide and maleimide derivatives such as N-ethylmaleimide are suitable.

As the SH reagent used in the present invention, the maleimide compound represented by General Formula [1] or 2-iodoacetamide is preferable.

As an amount to be added for the enzymatic coupling reaction of the second process, an amount equivalent to or up to 20 times that of the reducing agent used in the reaction of the first process is suitable.

Reactions of the first process and the second process can be caused at the same time. GDP or UDP is measured when reactions of the first process and the second process can be caused at the same time. When the CMP is measured, since addition of the reducing agent and a deactivation operation thereof are necessary, it is not preferable to perform the first process and the second process at the same time.

<Method for screening for an activity control agent of an enzyme>

When the method for measuring enzyme activities of the present invention described above is used to screen for an activity control agent of a glycosyltransferase or an ADP-producing enzyme such as a kinase, a test compound is preferably added to wells using a microplate. In the presence of the test compound and in the absence thereof (as a control group), a reaction of the glycosyltransferase or the ADP-producing enzyme such as a kinase is caused. Then, the enzymatic coupling reaction is caused to measure fluorescence as described above, and thus activities of the glycosyltransferase or the ADP-producing enzyme such as a kinase are quantified.

When activities of the obtained glycosyltransferase or ADP-producing enzyme such as a kinase are compared according to the presence and absence of the test compound, it is possible to determine an activity control action on the glycosyltransferase or the ADP-producing enzyme such as a kinase of the test compound.

In addition, when only the enzymatic coupling reaction is caused in the presence of the test compound without causing a reaction of the glycosyltransferase or the ADP-producing enzyme such as a kinase, it is possible to know

whether the test compound influenced the enzymatic coupling reaction, and confirm whether activities of the test compound influence the glycosyltransferase or the ADP-producing enzyme such as a kinase.

In fluorescence produced during the enzymatic coupling reaction according to the present invention, since an excitation wavelength is about 540 nm and a fluorescence wavelength is about 590 nm, a related wavelength band is influenced less by absorption or autofluorescence of ultraviolet and visible regions of the test compound, which is advantageous.

A method for screening for an activity control agent of a glycosyltransferase, which is one embodiment of the method for screening for an activity control agent of an enzyme, includes a glycosyltransferase reaction process in which a glycosyltransferase reaction is caused in the presence or absence of the test compound, a first process in which GDP or UDP produced during the glycosyltransferase reaction is reacted with an NDP kinase in the presence of ATP, or CMP produced during the glycosyltransferase reaction is reacted with a CMP kinase in the presence of ATP, and ADP corresponding to an amount of the GDP, UDP or CMP is produced, a second process in which the ADP is quantified according to fluorescence caused by the enzymatic coupling reaction using glucose, an ADP-dependent hexokinase, glucose-6-phosphate dehydrogenase, a diaphorase, NADP and resazurin, and a comparison process in which activities of the glycosyltransferase are compared according to the presence or absence of the test compound and an activity control action on the glycosyltransferase of the test compound is determined.

In the method for screening for an activity control agent of a glycosyltransferase, which is one embodiment of the method for screening for an activity control agent of an enzyme, preferably, the first process is a process in which the CMP produced during the glycosyltransferase reaction is reacted with the CMP kinase in the presence of the ATP and the reducing agent, and thus ADP corresponding to the amount of GDP, UDP or CMP is produced, and the second process is a process in which fluorescence caused by the enzymatic coupling reaction in the presence of the SH reagent is quantified.

In the method for screening for an activity control agent of a glycosyltransferase, which is one embodiment of the method for screening for an activity control agent of an enzyme, the glycosyltransferase is preferably at least one type selected from among the group including fucosyltransferases, mannosyltransferases, glucosyltransferases, galactosyltransferases, glucuronosyltransferases, xylosyltransferases, N-acetylglucosaminyltransferases, N-acetylgalactosaminyltransferases, and sialyltransferases.

In a method for screening for an activity control agent of an ADP-producing enzyme, which is one embodiment of the method for screening for an activity control agent of an enzyme, an ADP-producing enzymatic reaction is caused in the presence or absence of the test compound. Then, the ADP produced during the ADP-producing enzymatic reaction is reacted with glucose, an ADP-dependent hexokinase, glucose-6-phosphate dehydrogenase, a diaphorase, NADP and resazurin in the presence of the SH reagent, the fluorescence of resulting resorufin is measured, activities are compared according to the presence or absence of the test compound, and an activity control action of the test compound on the ADP-producing enzyme is determined.

In the method for screening for an activity control agent of an ADP-producing enzyme, which is one embodiment of the method for screening for an activity control agent of an enzyme, the ADP-producing enzyme is preferably at least one type selected from among the group including kinases, ATPases, nitrogenases, tetrahydrofolate synthases, acetyl-CoA carboxylase, pyruvate carboxylase, and glutathione synthase.

In the method for screening for an activity control agent of an ADP-producing enzyme, which is one embodiment of the method for screening for an activity control agent of an enzyme, the SH reagent is preferably N-ethylmaleimide or iodoacetamide.

<ADP measurement kit>

The present invention provides an ADP measurement kit including glucose, an ADP-dependent hexokinase, glucose-6-phosphate dehydrogenase, a diaphorase, NAD and/or NADP, resazurin and an SH reagent.

As the SH reagent included in the ADP measurement kit of the present invention, the same as that described in the method for measuring ADP is used. Therefore, description thereof will be omitted.

It is possible to measure ADP in a sample in a simple and easy manner using the ADP measurement kit of the present invention.

The ADP measurement kit may include the reducing agent. When the method for measuring ADP of the present invention is performed in the presence of the reducing agent, dithiothreitol (DTT), 2-mercaptoethanol or TCEP is suitable as the reducing agent.

<ADP-producing enzyme activity measurement kit>

The present invention provides an ADP-producing enzyme activity measurement kit including glucose, an ADP-dependent hexokinase, glucose-6-phosphate dehydrogenase, a diaphorase, NAD and/or NADP, resazurin and an SH

reagent.

As the SH reagent included in the ADP-producing enzyme activity measurement kit of the present invention, the same as that described in the method for measuring activities of an ADP-producing enzyme is used. Therefore, description thereof will be omitted.

It is possible to measure activities of an ADP-producing enzyme in a simple and easy manner using the ADP-producing enzyme activity measurement kit of the present invention.

The ADP-producing enzyme activity measurement kit may include the reducing agent. When the method for measuring activities of an ADP-producing enzyme of the present invention is performed in the presence of the reducing agent, dithiothreitol (DTT), 2-mercaptoethanol or TCEP is suitable as the reducing agent.

<Glycosyltransferase activity measurement kit>

The present invention provides a transferase activity measurement kit including a first solution containing ATP and NMP kinase, an NDP kinase or a CMP kinase, and a second solution containing glucose, an ADP-dependent hexokinase, glucose-6-phosphate dehydrogenase, a diaphorase, NAD and/or NADP, resazurin, and an the SH reagent.

As one embodiment, in the glycosyltransferase activity measurement kit of the present invention, it is preferable that the first solution further include a reducing agent.

For example, since enzyme activities of certain types of enzymes such as CMP kinases increase according to addition of the reducing agent, it is possible to increase enzyme activities of the CMP kinase when the first solution further includes the reducing agent. Further, in the transferase activity measurement kit of the present invention, since the second solution includes the SH reagent, it is possible to deactivate the reducing agent, it is possible to suppress reduction from resazurin to resorufin, and it is possible to suppress background of fluorescence from increasing.

As the SH reagent included in the glycosyltransferase activity measurement kit of the present invention, the same as that described in the method for measuring activities of glycosyltransferase is used. Therefore, description thereof will be omitted.

It is possible to measure activities of glycosyltransferase in a simple and easy manner using the glycosyltransferase activity measurement kit of the present invention.

[Examples]

While the present invention will be described in further detail below with reference to examples, the present invention is not limited to the following examples.

(Example 1)

Calibration curves of GDP and UDP using a method of the present invention.

Calibration curves of GDP and UDP using reactions of the first process and the second process according to the present invention were created.

As materials, GDP (catalog number 078-04741 commercially available from Wako Pure Chemical Industries, Ltd.), UDP (catalog number 212-00861 commercially available from Wako Pure Chemical Industries, Ltd.), ATP (catalog number 18-16911 commercially available from Wako Pure Chemical Industries, Ltd.), an NDP kinase derived from baker's yeast (catalog number N0379 commercially available from Sigma-Aldrich), glucose (catalog number 045-31162 commercially available from Wako Pure Chemical Industries, Ltd.), an ADP-dependent hexokinase (catalog number T-93 commercially available from Asahi Kasei Pharma Corporation., derived from Thermococcus litoralis), G-6-P dehydrogenase (catalog number 46857003 commercially available from Oriental Yeast Co., Ltd.), a diaphorase (catalog number B1D111 commercially available from Unitika Ltd.), NADP (catalog number 44290000 commercially available from Oriental Yeast Co., Ltd.), and resazurin (catalog number 191-07581 commercially available from Wako Pure Chemical Industries, Ltd.) were used. The GDP and UDP were dissolved in a buffer solution C (100 mM Tris-HCl (pH 7.5), and 5 mM $MgCl_2$) to prepare a 0 to 50 μM solution. The mixed solution for enzymatic coupling was prepared with the following composition.

Mixed solution A for a first process

| | |
|---|---|
| ATP | 200 μM |
| NDP kinase | 2 μg/ml |

(continued)

| | |
|---|---|
| Buffer solution composition | C |

Mixed solution B for a second process

| | |
|---|---|
| Glucose | 2 mM |
| ADP-dependent hexokinase | 2 U/ml (38 μg/ml) |
| G-6-P dehydrogenase | 2 U/ml (2.6 μg/ml) |
| Diaphorase | 2 U/ml (1.1 μg/ml) |
| NADP | 200 μM |
| Resazurin | 100 μM |
| Buffer solution composition | C |

A 0 to 50 μM GDP or UDP solution (4 μl) and the mixed solution A for the first process (4 μl) were added to a 384-well microplate (a small volume, an unbound type, and commercially available from Greiner under catalog number 784900), and incubated for 1 hour at 25 °C. The mixed solution B for the second process (8 μl) was added thereto and the result was incubated for 1 hour at 25 °C.

A plate reader PHERAstar commercially available from BMG Labtech was used to measure fluorescence at an excitation wavelength of 540 nm and a fluorescence wavelength of 590 nm. The results are shown in Fig. 2. Accordingly, a fluorescence intensity according to a concentration of the GDP or UDP was confirmed, and it was confirmed that both nucleotides can be quantified using a method of the present invention.

(Example 2)

Calibration curve of CMP using a method of the present invention

A calibration curve of CMP using reactions of the first process and the second process according to the present invention was created.

As materials, CMP (catalog number 034-05361 commercially available from Wako Pure Chemical Industries, Ltd.), a CMP kinase (human CMPK1, catalog number PKA-002 commercially available from Prospec), DTT (catalog number 040-29223 commercially available from Wako Pure Chemical Industries, Ltd.), and N-ethylmaleimide (catalog number 054-02061 commercially available from Wako Pure Chemical Industries, Ltd.) were used. The CMP was dissolved in a buffer solution F (100 mM Tris-HCl (pH 9), 13.5 mM $MgCl_2$, 150 mM KCl, and 0.1 % Triton X-100) to prepare a 0 to 40 μM solution. The mixed solution for enzymatic coupling was prepared with the following composition.

Since a reducing agent is required with the CMP kinase, DTT was used as the reducing agent in the first process, and N-ethylmaleimide was used as the SH reagent for deactivating the reducing agent in the second process.

Mixed solution D for a first process

| | |
|---|---|
| ATP | 200 μM |
| CMPK1 | 3 μg/ml |
| DTT | 4 mM |
| Buffer solution composition | F |

Mixed solution E for a second process

| | |
|---|---|
| Glucose | 2 mM |
| ADP-dependent hexokinase | 2 U/ml (38 μg/ml) |
| G-6-P dehydrogenase | 2 U/ml (2.6 μg/ml) |

(continued)

| | |
|---|---|
| Diaphorase | 2 U/ml (1.1 μg/ml) |
| NADP | 200 μM |
| Resazurin | 100 μM |
| N-ethylmaleimide | 20 mM |
| Buffer solution composition | C |

According to the same operations as those in Example 1, reactions of the first process and the second process were caused using the mixed solution for a CMP solution, and fluorescence was measured to obtain a calibration curve of the CMP. The results are shown in Fig. 3. A fluorescence intensity according to a concentration of the CMP was observed, and it was confirmed that the CMP can be quantified using a method of the present invention.

(Example 3)

Change of fluorescence development over time

40 μM GDP or UDP was used to cause the same reactions as those in Example 1. Then, the plate was removed intermittently only during the reaction of the second process, and fluorescence was measured. In addition, 40 μM CMP was used to cause the same reactions as those in Example 2, the plate was removed intermittently only during the reaction of the second process, and fluorescence was measured. The results are shown in Fig. 4. Accordingly, when the GDP or UDP was quantified, the reaction of the second process was completed within 25 minutes, and a fluorescence value was stable until at least the 120 minute mark. Therefore, it was seen that, when the CMP was quantified, the reaction of the second process was almost completed within 25 minutes, and there was almost no change in a fluorescence value from at least the 60 minute mark until the 120 minute mark.

Therefore, it was seen that a stable measurement result was obtained if fluorescence was measured from 25 minutes to 120 minutes after the second process started when the GDP or UDP was quantified, and measured from 60 minutes to 120 minutes after the second process started when the CMP was quantified.

(Example 4)

Measurement of activities of a glycosyltransferase (1)

Activities of a fucosyltransferase serving as the glycosyltransferase were measured using a method of the present invention. Human FUT7 (catalog number 6409-GT commercially available from R&D Systems) was used as the fucosyltransferase. CDP-fucose (catalog number 7229 commercially available from YAMASA Corporation) and fetuin (derived from fetal bovine serum, catalog number F3004 commercially available from Sigma-Aldrich) were used as substrates.

100 μM GDP-fucose (2.5 μl), 6 mg/ml fetuin, and 0 to 4 μg/ml FUT7 (2.5 μl) were added to a 384-well microplate (a buffer solution included 50 mM Tris-HCl (pH 7.5), 5 mM $MgCl_2$, 10 mM $MnCl_2$, and 0.1% Triton X-100), covered with a plate seal (catalog number 1-6774-01 commercially available from AS ONE corporation) and incubated for 1 hour at 37 °C. After being cooled to room temperature, the mixed solution A for the first process (5 μl) was added to the result and incubated for 1 hour at 25 °C. The mixed solution B for the second process (10 μl) was added to the result, and incubated for 30 minutes at 25 °C. Fluorescence thereof was measured similarly to Example 1. The results are shown in Fig. 5. A fluorescence value according to an enzyme concentration was confirmed.

(Example 5)

Measurement of activities of a glycosyltransferase (2)

Activities of a galactosyltransferase serving as the glycosyltransferase were measured using a method of the present invention.

Human B4GalT1 (catalog number 3609-GT commercially available from R&D Systems) was used as the galactosyltransferase. UDP-galactose (catalog number 195905 commercially available from MP Biomedicals) and N-acetyl-D-glucosamine (catalog number 013-12181 commercially available from Wako Pure Chemical Industries, Ltd.) were used as substrates. 100 μM UDP-galactose (2.5 μl), 10 mM N-acetyl-D-glucosamine, and 0 to 0.3 μg/ml B4GalT1 (2.5 μl) were added to a 384-well microplate (a buffer solution included 50 mM Tris-HCl (pH 7.5), 5 mM $MgCl_2$, 10 mM $MnCl_2$,

and 0.1 % TritonX-100), covered with a plate seal, and incubated for 1 hour at 37 °C.

After being cooled to room temperature, a mixed solution (15 μl) including the mixed solution A for the first process (5 μl) and the mixed solution B for the second process (10 μl) was added to the result, and incubated for 1 hour at 25 °C. Fluorescence thereof was measured similarly to Example 1. The results are shown in Fig. 6. A fluorescence value according to an enzyme concentration was confirmed, and it was seen that measurement was possible when the first process and the second process were performed at the same time.

(Example 6)

Measurement of activities of a glycosyltransferase (3)

Activities of a sialyltransferase serving as the glycosyltransferase were measured using a method of the present invention. Human ST6Gal1 (catalog number 5924-GT commercially available from R&D Systems) was used as the sialyltransferase. CMP-sialic acid (catalog number 233264 commercially available from Calbiochem) and N-acetyl-D-galactosamine (catalog number A7791 commercially available from Sigma Aldrich) were used as substrates.

100 μM CMP-sialic acid (2.5 μl), 500 μM N-acetyl-D-galactosamine, and 0 to 10 μg/ml ST6Gal1 (2.5 (μl) were added to a 384-well microplate (a buffer solution included 25 mM Tris-HCl (pH 7.5), 5 mM $MgCl_2$, 5 mM $MnCl_2$, 150 mM NaCl, and 0.1 % TritonX-100), covered with a plate seal, and incubated for 1 hour at 37 °C. After being cooled to room temperature, the mixed solution D for the first process (5 μl) was added to the result, covered with a plate seal, and incubated for 1 hour at 37 °C. After being cooled to room temperature, the mixed solution E for the second process (10 μl) was added to the result and incubated for 1 hour at 25 °C. Fluorescence thereof was measured similarly to Example 1. The results are shown in Fig. 7. A fluorescence value according to an enzyme concentration was confirmed.

(Example 7)

Measurement of glycosyltransferase inhibitory activities

Enzyme inhibitory activities of gallic acid on human FUT7 were measured using a method of the present invention. Gallic acid is a compound that has been reported to have inhibitory activities on human FUT7 (refer to Arch. Biochem. Biophys, Vol. 425, No.1, pp.51-57, 2004).

When enzyme activities of FUT7 were measured using the method described in Example 4, an inhibitory effect of gallic acid on FUT7 was studied according to the same operations as in Example 4 except that a reaction of FUT7 (a concentration of 1 μg/ml in a transglucosylation solution) was caused in the presence of gallic acid. A group containing no gallic acid was set as a control (an inhibition rate of 0%), a group containing no FUT7 was set as a control (an inhibition rate of 100%), and inhibition rates of gallic acid of respective concentrations of FUT7 were calculated.

In addition, the same operations were performed using 50 μM GDP instead of 100 μM GDP-fucose, and an influence of enzymatic coupling itself on gallic acid was studied. In addition, an amount of GDP produced was quantified by HPLC analysis using some of the reaction solution after the FUT7 enzymatic reaction, and an inhibitory activity was determined by an HPLC method.

HPLC conditions were as follows: column: YMC-Triart C18 (commercially available from Ymc Corporation, particle size: 5 μm, and diameter: 4.6 mm×length: 150 mm), eluate: 50 mM $KH_2PO_4$-$K_2HPO_4$ (10:1), flow rate: 1 ml/min, and detection: UV 260 nm. The measurement results are shown in Fig. 8.

It was seen that the inhibitory activity of gallic acid measured using a method of the present invention matched the inhibitory activity measured by HPLC (an IC50 value is about 10 μM). Further, gallic acid did not inhibit the enzymatic coupling reaction itself at all, and the inhibitory activity measured using enzymatic coupling was confirmed to be caused by inhibition of FUT7.

In addition, in Arch. Biochem. Biophys, vol. 425, No.1, pp. 51-57, 2004, an IC50 value of gallic acid on human FUT7 was reported as 5.4 μM. The IC50 value measured in Example 7 was confirmed to be close to the value in the literature.

As described above, it was seen that glycosyltransferase inhibitory activities can be accurately measured using the method of the present invention.

(Example 8)

Glycosyltransferase activity measurement kit (1)

An activity measurement kit for a fucosyltransferase, mannosyltransferase, glucosyltransferase, galactosyltransferase, glucuronosyltransferase, xylosyltransferase, N-acetylglucosaminyltransferase, or N-acetylgalactosaminyltransferase was prepared with the following composition.

Mixed solution for a first process

| | |
|---|---|
| ATP | 200 μM |
| NDP kinase | 2 μg/ml |
| Buffer solution composition | 100 mM Tris-HCl (pH 7.5), 5 mM $MgCl_2$ |

Mixed solution for a second process

| | |
|---|---|
| Glucose | 2 mM |
| ADP-dependent hexokinase | 2 U/ml (38 μg/ml) |
| G-6-P dehydrogenase | 2 U/ml (2.6 μg/ml) |
| Diaphorase | 2 U/ml (1.1 μg/ml) |
| NADP | 200 μm |
| Resazurin | 100 μM |
| Buffer solution composition | 100 mM Tris-HCl (pH 7.5), 5 mM $MgCl_2$ |

(Example 9)

Glycosyltransferase activity measurement kit (2)

A sialyltransferase activity measurement kit was prepared with the following composition.

Mixed solution for a first process

| | |
|---|---|
| ATP | 200 μM |
| CMPK1 | 3 μg/ml |
| DTT | 4 mM |
| Buffer solution composition | 100 mM Tris-HCl (pH 9), 13.5 mM $MgCl_2$, 150 mM |

KCl, and 0.1% TritonX-100

Mixed solution for a second process

| | |
|---|---|
| Glucose | 2 mM |
| ADP-dependent hexokinase | 2 U/ml (38 μg/ml) |
| G-6-P dehydrogenase | 2 U/ml (2.6 μg/ml) |
| Diaphorase | 2 U/ml (1.1 μg/ml) |
| NADP | 200 μM |
| Resazurin | 100 μM |
| N-ethylmaleimide | 20 mM |
| Buffer solution composition | 100 mM Tris-HCl (pH 7.5), and 5 mM $MgCl_2$ |

(Example 10)

Calibration curve of ADP according to a method of the present invention

ADP (catalog number 45120000 commercially available from Oriental Yeast Co., Ltd.) was dissolved in a buffer solution C (100 mM Tris-HCl (pH 7.5), and 5 mM MgCl2) to prepare a 0 to 37.5 μM solution. The mixed solution for enzymatic coupling was prepared with the following composition. When N-ethylmaleimide was added, 20 mM N-ethylmaleimide was added to the mixed solution for enzymatic coupling.

Mixed solution composition for enzymatic coupling

| | |
|---|---|
| D-glucose | 2 mM |
| ADP-dependent hexokinase | 2 U/ml (38 μg/ml) |
| G-6-P dehydrogenase | 2 U/ml (2.6 μg/ml) |
| Diaphorase | 2 U/ml (1.1 μg/ml) |
| NADP | 200 μM |
| Resazurin | 100 μM |
| N-ethylmaleimide | 20 mM or no addition |
| Buffer solution composition | C |

A 0 to 37.5 μM ADP solution (7.5 μl) (a buffer solution composition was C) and a mixed solution for enzymatic coupling (7.5 μl) were added to a 384-well microplate (a small volume, an unbound type, and commercially available from Greiner under catalog number 784900) and incubated for 1 hour at 25 °C in darkness.

Then, a fluorescence intensity was measured using a microplate reader PHERAstar commercially available from BMG Labtech at an excitation wavelength of 540 nm, and a fluorescence wavelength of 590 nm. The results are shown in Fig. 9. An ADP-concentration-dependent fluorescence intensity was confirmed, and it was seen that good linearity could be obtained up to an ADP concentration of 25 μM.

In addition, even when N-ethylmaleimide was included, almost no change was observed in the fluorescence value. Accordingly, it was seen that fluorescence of ADP can be quantified using the enzymatic coupling reaction in the presence of the SH reagent according to the present invention.

(Example 11)

Study of stability of fluorescence development using enzymatic coupling of ADP.

25 μM ADP was used to cause the same reaction as in Example 10. However, during the enzymatic coupling reaction, the microplate was removed intermittently and fluorescence was measured. The results are shown in Fig. 10. Accordingly, it was seen that the enzymatic coupling reaction in ADP quantification was completed within 10 to 20 minutes, and the fluorescence value was stable for 2 hours thereafter.

(Example 12)

Calibration curve of ADP in the presence of the reducing agent DTT

A calibration curve of ADP in the presence of DTT was measured similarly to Example 10. The results are shown in Fig. 11. Background of fluorescence increased according to the presence of DTT. However, it was confirmed that, when 20 mM N-ethylmaleimide (NEM) was mixed in advance in the mixed solution for enzymatic coupling, an increase in the background was suppressed and the ADP had a good quantitative property. When the result is represented as a count ratio of signal/background (an S/B ratio, a higher value indicates higher sensitivity) serving as a specificity index of detection sensitivity of an assay system, the S/B ratio exhibited a high value of 57.4 as long as no DTT was included when 20 μM ADP was quantified. However, when 2 mM DTT was included, the S/B ratio was reduced to 4.2, less than 1/10 of that before the DTT was included. However, when 20 mM N-ethylmaleimide was added, the S/B ratio was 36.7, which was increased to about 9 times the value obtained when no N-ethylmaleimide was added.

(Example 13)

Calibration curve of ADP in the presence of iodoacetamide

In Example 12, a calibration curve of ADP was measured according to the same operations as in Example 12 except that 8 mM iodoacetamide (IAA) was used instead of 20 mM N-ethylmaleimide. The results are shown in Fig. 12. When 20 μM ADP was quantified, the S/B ratio was 3.7 in the presence of 2 mM DTT, and there was about a fivefold increase to 18.6 when 8 mM iodoacetamide was used. Accordingly, it was confirmed that, when iodoacetamide was mixed in advance in the mixed solution for enzymatic coupling, specificity of measurement increased.

(Example 14)

Measurement of activities of a kinase

Human CMPK1 (CMP kinase1, catalog number pKa-002-b commercially available from Prospec) was used as a kinase requiring a reducing agent, and a dependence of CMPK1 on DTT was studied first.

400 μM ATP (10 μl), 80 μM CMP (a buffer solution composition included 100 mM Tris-HCl (pH 9), 13.5 mM $MgCl_2$, 150 mM KCl, and 0.1% TritonX-100), a 0 to 8 mM DTT aqueous solution (5 μl) and 4 μg/ml CMPK1 (5 μl) (a buffer solution composition included 100 mM Tris-HCl (pH 9), 13.5 mM $MgCl_2$, 150 mM KCl, and 0.1 % TritonX-100) were added to a micro-centrifuge tube (1.5 ml in volume), and incubated for 1 hour at 37 °C. The reaction solution (15 μl) was input to HPLC to quantify ADP and CDP (cytosine 5'-diphosphate) produced during a CMPK1 enzymatic reaction. HPLC conditions were as follows: column: YMC-Triart C18 (commercially available from Ymc Corporation, particle size: 5 μm, and diameter: 4.6 mm×length: 150 mm), eluate: 50 mM $KH_2PO_4$-$K_2HPO_4$ (10:1), flow rate: 1 ml/min, and detection: UV 265 nm. The results are shown in Fig. 13.

It was confirmed that enzyme activities of CMPK1 depended on a DTT concentration, and the same molar quantities of CDP and ADP were produced. Accordingly, it was seen that CMPK1 showed sufficient activities as long as the DTT concentration was equal to or greater than 1 mM.

Next, enzyme activities of CMPK1 in the presence of DTT were measured using a method of the present invention. 0 or 40 μM CMP (2.5 μl), 200 μM ATP (2.5 μl), 0 to 3 μg/ml CMPK1, 4 mM DTT (a buffer solution composition included 100 mM Tris-HCl (pH 9), 13.5 mM $MgCl_2$, 150 mM KCl, and 0.1% Triton X-100) were added to a 384-well microplate, covered with a plate seal (catalog number 1-6774-01 commercially available from AS ONE corporation), and incubated for 1 hour at 37 °C. After being cooled to room temperature, the mixed solution for enzymatic coupling (5 μl) (a composition was the same as in Example 10) was added to the result, and incubated at 25 °C. Fluorescence was measured five times in total, at 0 minutes, 15 minutes, 30 minutes, 60 minutes, and 120 minutes after incubation started, and the S/B ratio was determined similarly to Example 12. Measurement results of the S/B ratio when a CMPK1 concentration was 3 μg/ml are shown in Fig. 14.

When the enzymatic coupling reaction was caused without N-methyl maleimide, the S/B ratio was 3 to 4. On the other hand, the S/B ratio was 13 to 14 when N-ethylmaleimide was included for an assay. It was seen that, when enzymatic coupling was caused in the presence of the SH reagent, a quantitative property of kinase activities was significantly improved.

In addition, concentration-dependent quantification results of CMPKin the presence of N-ethylmaleimide for a fluorescence measurement time of 60 minutes are shown in Fig. 15. Kinase activities according to a CMPK1 enzyme concentration were confirmed.

(Example 15)

Measurement of kinase inhibitory activities

Enzyme inhibitory activities of $Ap_5A$ ($P^1$,$P^5$-Di(adenosine-5')pentaphosphate) in which inhibitory activities of dictyostelium (Dictyostelium discoideum) on CMP kinase have been reported (J. Biol. Chem, Vol. 265, No. 11, pp. 6339-6345, 1990) on CMPK1 were measured using a method of the present invention. A 0 to 3 mM $Ap_5A$ (1 μl) (catalog number D4022 commercially available from Sigma-Aldrich) solution and 50 μM CMP (2 μl) were added to a 384-well microplate, and 200 μM ATP (2 μl), 3 μg/ml CMPK1, and 4 mM DTT were added thereto (a buffer solution composition included 100 mM Tris-HCl (pH 9), 13.5 mM $MgCl_2$, 150 mM KCl, and 0.1% TritonX-100). The result was covered with a plate seal and incubated for 1 hour at 37 °C. Then, the result was cooled at room temperature, and the mixed solution for enzymatic coupling (5 μl) containing 20 mM N-ethylmaleimide was added and incubated for 60 minutes at 25 °C in darkness. Then, fluorescence was measured using a plate reader. A group containing no $Ap_5A$ was set as a control (an inhibition rate of 0%), a group containing no CMPK1 was set as a control (an inhibition rate of 100%), and inhibition

rates of $Ap_5A$ of respective concentrations on CMPK1 were calculated.

In addition, the same operations were performed using 25 μM ADP instead of 50 μM CMP, and an influence of $Ap_5A$ on enzymatic coupling itself was studied. In addition, some of the reaction solution after the kinase reaction of CMPK1 was used, an amount of produced CDP was measured by the HPLC analysis method described in Example 14, and an inhibitory activity was determined by an HPLC method. The results are shown in Fig. 16.

It was seen that an inhibitory activity of $Ap_5A$ on CMPK1 measured using a method of the present invention matched an inhibitory activity measured by the HPLC method. Further, it was confirmed that $Ap_5A$ did not inhibit the enzymatic coupling reaction itself at all, and the inhibitory activity measured by enzymatic coupling was caused by inhibition of CMPK1.

As described above, it was seen that kinase inhibitory activities can be accurately measured using the method of the present invention.

(Example 16)

Kinase activity measurement kit

A kinase activity measurement kit was prepared with the following composition.

Composition of the kinase activity measurement kit

| | |
|---|---|
| D-glucose | 2 mM |
| ADP-dependent hexokinase | 2 U/ml (38 μg/ml) |
| G-6-P dehydrogenase | 2 U/ml (2.6 μg/ml) |
| Diaphorase | 2 U/ml (1.1 μg/ml) |
| NADP | 200 μM |
| Resazurin | 100 μM |
| N-ethylmaleimide | 20 mM |
| Buffer solution composition | 100 mM Tris-HCl (pH 7.5), 5 mM $MgCl_2$ |

(Example 17)

Measurement of ATPase activities

Activities of an ATPase contaminating a commercially available kinase were measured using a method of the present invention. 400 μM ATP (2.5 μl) and 0 to 16 μg/ml human UMP kinase (2.5 μl) (catalog number E-NOV-4 commercially available from Novocib) were added to a 384-well microplate (a buffer solution composition included 50 mM Tris-HCl (pH 7.5), 5 mM $MgCl_2$, 50 mM NaCl, 1 mM DTT, 200 μM $Na_3VO_4$, and 0.1% TritonX-100), and incubated for 1 to 2 hours at 30 °C. The kit solution (5 μl) described in Example 16 was added thereto and incubated for 1 hour at 25 °C. Then, fluorescence was measured using a plate reader similarly to Example 10. In addition, the enzymatic reaction solution before enzymatic coupling was analyzed under the HPLC conditions used in Example 7, and ADP was directly quantified by HPLC. The results are shown in Figs. 17A and 17B. It was seen that, in commercially available human UMP kinase (UMPK) with no phosphate acceptor, ADP was produced from ATP showing ATPase activities, the ATPase activities could be quantified using the method of the present invention, and the measurement results matched each other. Also, since human UMP kinase did not phosphorylate proteins, there was no autophosphorylation activity. The ATPase activities shown herein can be considered to have been caused by the remaining ATPase that was not removed in an enzyme purification process.

(Example 18)

Screening for a glycosyltransferase (galactosyltransferase) inhibitor

As a compound library, a LOPAC 1280 (trademark) library (commercially available from Sigma-Aldrich) including 1280 types of known biologically active substances was used. Compounds (dissolved at a concentration of 1 mM in dimethyl sulfoxide (DMSO)) of the LOPAC 1280 (trademark) library were added to a 384-well microplate at 320 compounds per plate, and were dispensed at a volume of 50 nl using a trace dispenser (Echo 555 commercially available from Labcyte).

10 mM N-acetyl-D-glucosamine (2.5 μl), 100 μM UDP-galactose, and 0.05 μg/ml human B4GalT1 (2.5 μl) were added thereto (a buffer solution included 50 mM Tris-HCl (pH 7.5), 5 mM $MgCl_2$, 10 mM $MnCl_2$, and 0.02% TritonX-100) using a continuous dispenser (Multidrop combi commercially available from Thermo Fisher Scientific), and covered with a plate seal, and incubated for 1 hour at 37 °C (a concentration of the library compounds in the reaction solution was 10 μM).

A well to which DMSO was added instead of the library compounds was set as a control well (a reaction rate of 100%), and a well to which no enzyme was added was set as a control well (a reaction rate of 0%) (both controls were provided in 16 wells per plate). After being cooled to room temperature, the mixed solution A for the first process (5 μl) was added and incubated for 1 hour at 25 °C, and the mixed solution B for the second process (10 μl) was added and further incubated for 1 hour at 25 °C. Then, fluorescence was quantified.

On the other hand, in the same manner as described above, the library compounds and DMSO were added to plates, plates to which 20 μM UDP was added at 5 μl instead of the substrate solution and the enzyme solution were prepared, reactions of the first process and the second process were similarly caused, and an influence on a UDP quantification reaction itself was studied. In this case, the control well (a reaction rate of 0%) was a well to which UDP (20 μM) was not added. In both assays, fluorescence quantification values of control wells having reaction rates of 100% and 0%, and a fluorescence quantification value of a well in which one of the library compounds was included were set as a, b, and c, respectively, and the inhibition rate was computed by the following equation.

[Equation 1]

$$\text{inhibition rate (\%)} = (1-(c-b)/(a-b))\times 100$$

The measurement results of the 1280 library compounds are shown in Figs. 18A to 18C. When a B4GalT1 reaction was caused, the number of compounds whose inhibition rates were 50% or more was 24 (Fig. 18A).

On the other hand, in a UDP quantification assay, the number of compounds whose inhibition rates were 50% or more was 10 (Fig. 18B). By computing a difference between inhibition rates of the assays (A-B), it was possible to estimate a net inhibition rate of the B4GalT1 reaction. The number of compounds whose differences were 50% or more was 12 (Fig. 18C).

Therefore, according to such screening, it was seen that 12 compounds that inhibit human galactosyltransferase B4GalT1 can be obtained from the LOPAC 1280 (trademark) library, and the present invention is beneficial as a screening method having a high throughput.

In addition, in screening methods of the related art, a well unit price is about 100 yen per well. On the other hand, in the screening method of the present invention, a well unit price is less expensive at 2 to 8 yen per well.

Therefore, it was confirmed that the screening method of the present invention was excellent in view of costs.

(Example 19)

Quantification of ADP in the presence ofmaleimides or iodoacetamide

ADP (catalog number 019-25091 commercially available from Wako Pure Chemical Industries, Ltd.) was dissolved to a concentration of 10 mM in pure water, and an ADP solution (pH 7) was prepared using 5 M sodium hydroxide (catalog number 196-05375 commercially available from Wako Pure Chemical Industries, Ltd.).

DTT (catalog number 040-29223 commercially available from Wako Pure Chemical Industries, Ltd.) was prepared to a concentration of 1 M in pure water, and a DTT solution was prepared.

A 10 mM ADP solution, a 10 mM ATP solution (V915B commercially available from Promega Corporation), and a 1 M DTT solution were diluted in a buffer solution 1 (40 mM Tris-HCl (pH 7.5), 20 mM $MgCl_2$, and 0.01% BSA). Solutions 1 to 6 having the compositions described in the following Table 1 were prepared.

[Table 1]

| | Concentration of DTT in buffer solution 1 | | |
|---|---|---|---|
| | DTT 0 mM | DTT 3 mM | DTT 6 mM |

(continued)

| | | Concentration of DTT in buffer solution 1 | | |
|---|---|---|---|---|
| | | DTT | DTT | DTT |
| Concent ration of ADP and ATP in buffer solution 1 | 0 μM ADP solution (0 μM ADP+100 μM ATP) | Solution 1 | Solution 3 | Solution 5 |
| | 20 μM ADP solution (20 μM ADP and 80 μM ATP) | Solution 2 | Solution 4 | Solution 6 |

As the SH reagent, maleimide (catalog number 133-13111 commercially available from Wako Pure Chemical Industries, Ltd.), N-ethylmaleimide (catalog number 058-02061 commercially available from Wako Pure Chemical Industries, Ltd.), and iodoacetamide (IAA) (catalog number 095-02151 commercially available from Wako Pure Chemical Industries, Ltd.) were prepared to a concentration of 800 mM in dimethyl sulfoxide (catalog number 041-29351 commercially available from Wako Pure Chemical Industries, Ltd.). N-(2-sulfoethyl)maleimide (catalog number SC-207907 commercially available from Santa Cruz Biotechnology, Inc.) was prepared to a concentration of 250 mM in pure water.

Mixed solutions for enzymatic coupling were prepared to have the compositions described in the following Table 2. When the SH reagent was used in the mixed solution for enzymatic coupling, the SH reagent was added to 40 mM.

[Table 2]

| Enzymatic coupling solution | | | |
|---|---|---|---|
| Solution A | D-glucose (2 mM) ADP-dependent hexokinase (2 U/ml (37 | | Without SH reagent |
| Solution B | μg/ml)) G-6-P dehydrogenase (2 U/ml (2.3 | | 40 mM maleimide |
| Solutio nC | μg/ml)) Diaphorase (2 U/ml (1.2 μg/ml)) | + | 40 mM N-ethylmaleimide |
| Solution D | NADP (5 μM) Resazurin (60 μM) | | 40 mM N-(2-sulfoethyl)maleimide |
| Solution E | Tris-HCl (pH 7.5) (100 mM) $MgCl_2$ (10 mM) 0.01% BSA 0.05% TritonX-100 | | 40 mM IAA |

5 μl each of solutions 1 to 6 and mixed solutions for enzymatic coupling of solutions A to E was added to a 384-well microplate (a small volume, an unbound type, and commercially available from Greiner under catalog number 784900) as shown in the following Table 3, and incubated for 1 hour at 25 °C.

[Table 3]

| | Solution 1 | Solution 2 | Solution 3 | Solution 4 | Solution 5 | Solution 6 |
|---|---|---|---|---|---|---|
| Solution A | Solution 1-A | Solution 2-A | Solution 3-A | Solution 4-A | Solution 5-A | Solution 6-A |
| Solution B | Solution 1-B | Solution 2-B | Solution 3-B | Solution 4-B | Solution 5-B | Solution 6-B |
| Solution C | Solution 1-C | Solution 2-C | Solution 3-C | Solution 4-C | Solution 5-C | Solution 6-C |
| Solution D | Solution 1-D | Solution 2-D | Solution 3-D | Solution 4-D | Solution 5-D | Solution 6-D |
| Solution E | Solution 1-E | Solution 2-E | Solution 3-E | Solution 4-E | Solution 5-E | Solution 6-E |

Then, a fluorescence intensity of the solutions shown in Table 3 was measured using a microplate reader Safire commercially available from TECAN at an excitation wavelength of 540 nm and a fluorescence wavelength of 590 nm.

The above experiment was performed twice. An average of obtained fluorescence intensities was obtained. The results are shown in Figs. 19A to 19C.

Background of fluorescence increased according to the presence of DTT. However, it was confirmed that, when 40 mM maleimides (maleimide, N-ethylmaleimide, and N-(2-sulfoethyl)maleimide) and 40 mM IAA were mixed in advance in the mixed solution for enzymatic coupling, an increase in the background was suppressed, and ADP had a good quantitative property. When the result is represented as a count ratio of signal/background (an S/B ratio, a higher value indicates higher sensitivity) serving as a specificity index of detection sensitivity of an assay system, the S/B ratio exhibited a high value of 23.3, as long as no DTT was included when 20 μM ADP was quantified. However, when 3 mM or 6 mM DTT was included, the S/B ratio was reduced to 4.0 or 2.9. However, if 40 mM maleimides (maleimide, N-ethylmaleimide,

or N-(2-sulfoethyl)maleimide) were added, the S/B ratio was 23.6, 23.8, and 24.0 when 3 mM DTT was added, and the S/B ratio was improved to 21.7, 22.5, and 21.0 when 6 mM DTT was added. If 40 mM IAA was added, the S/B ratio was 18.2 when 3 mM DTT was added, and the S/B ratio was improved to 13.3 when 6 mM DTT was added. Accordingly, it was seen that both maleimides and IAA had an effect of suppressing background of fluorescence, and particularly, maleimides had a high suppressing effect.

(Example 20)

Quantification of ADP in the presence of a reducing agent DTT, 2-mercaptoethanol, or TCEP

According to the same method as in Example 19, 6 mM DTT, 6 mM 2-mercaptoethanol (catalog number 135-07522 commercially available from Wako Pure Chemical Industries, Ltd.), and 6 mM TCEP (catalog number 209-19861 commercially available from Wako Pure Chemical Industries, Ltd.) were added as the reducing agents, and ADP was quantified.

TCEP was dissolved to a concentration of 1 M in pure water, prepared to have a pH of 7 using 5 M sodium hydroxide (catalog number 196-05375 commercially available from Wako Pure Chemical Industries, Ltd.), diluted to 6 mM, and then used.

ADP, ATP, and reducing agents were dissolved in a buffer solution (40 mM Tris-HCl (pH 7.5), 20 mM $MgCl_2$, and 0.01% BSA), and each of the reducing agents (6 mM), ADP 0 μM and a 20 μM solution (ADP 0 μM and ATP 100 μM, or ADP 20 μM and ATP 80 μM) was prepared. A mixed solution for enzymatic coupling had the same composition as in Example 19, and maleimides (maleimide, and N-ethylmaleimide) or IAA was added at a concentration of 40 mM.

The reducing agent at 6 mM (5 μl), 0 μM ADP, a 20 μM solution (a sum of ADP and ATP was 100 μM, and a buffer solution included 40 mM Tris-HCl (pH 7.5), 20 mM $MgCl_2$, and 0.01% BSA), and a mixed solution for enzymatic coupling (5 μl) were added to a 384-well microplate (a small volume, an unbound type, and commercially available from Greiner under catalog number 784900), and incubated for 1 hour at 25 °C.

Then, a fluorescence intensity was measured using a microplate reader Safire commercially available from TECAN at an excitation wavelength of 540 nm and a fluorescence wavelength of 590 nm.

The above experiment was performed three times. An average of obtained fluorescence intensities was obtained. The results are shown in Figs. 20A to 20C.

When 20 μMADP was quantified, if 6 mM DTT, 2-mercaptoethanol, and TCEP were included, the S/B ratio was 1.2, 14.0, and 3.4 when no SH reagent was added, the S/B ratio was 16.1, 19.1, and 19.4 when a maleimide was added, the S/B ratio was 18.2, 19.6, and 19.9 when N-ethylmaleimide was added, and the S/B ratio was improved to 9.0, 18.8, and 12.1 when IAA was added. Accordingly, it was seen that an effect of maleimides and IAA suppressing background of fluorescence was efficient when the reducing agents DTT, 2-mercaptoethanol, and TCEP were added, and particularly, maleimides had a high effect.

[Industrial Applicability]

Since it is possible to measure a fluorescence intensity or absorbance resulting from reduction from resazurin to resorufin with high sensitivity, the present invention can be widely applied in the fields of chemistry, pharmaceutics, biochemistry, and the like.

## Claims

1. A method for detecting fluorescence or absorbance comprising:

   reducing, by a diaphorase, resazurin to resorufin, in the presence of an SH reagent and NADH or NADPH, and measuring the resulting fluorescence intensity or absorbance.

2. The method for detecting fluorescence or absorbance according to Claim 1, wherein the SH reagent is a maleimide compound represented by the following General Formula [1] or 2-iodoacetamide,

[Chemical Formula 1]

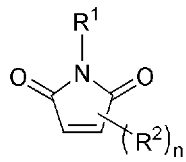
[1]

(where, in Formula [1],

$R^1$ represents a hydrogen atom, a hydroxyl group, a linear or branched alkyl group that optionally has a substituent group and has 1 to 6 carbon atoms, a linear or branched alkoxy group that optionally has a substituent group and has 1 to 6 carbon atoms, a linear or branched hydroxyalkyl group that optionally has a substituent group and has 1 to 6 carbon atoms, a linear or branched sulfoalkyl group that optionally has a substituent group and has 1 to 6 carbon atoms, or an aryl group that optionally has a substituent group and has 6 to 10 carbon atoms,
$R^2$ represents a hydrogen atom, a hydroxyl group, a halogen atom, a linear or branched alkyl group that optionally has a substituent group and has 1 to 6 carbon atoms, a linear or branched alkoxy group that optionally has a substituent group and has 1 to 6 carbon atoms, a linear or branched hydroxyalkyl group that optionally has a substituent group and has 1 to 6 carbon atoms, or a linear or branched sulfoalkyl group that optionally has a substituent group and has 1 to 6 carbon atoms, and
n represents the number of $R^2$ and is 0 or 1).

**3.** A method for suppressing background comprising:

an operation in which, when a fluorescence intensity or absorbance caused by reactions of NADH or NADPH, resazurin and a diaphorase in the presence of a reducing agent is measured, the reactions are caused in the presence of an SH reagent.

**4.** The method for suppressing background according to Claim 3,
wherein the SH reagent is a maleimide compound represented by the following General Formula [1] or 2-iodoacetamide,

[Chemical Formula 2]

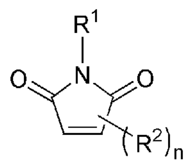
[1]

(where, in Formula [1], $R^1$, $R^2$, and n have the same meanings as described above).

**5.** A method for measuring ADP comprising:

a 2-1 process in which glucose is reacted with ADP and an ADP-dependent hexokinase to produce glucose-6-phosphate;
a 2-2 process in which the glucose-6-phosphate obtained in the 2-1 process is reacted with NAD or NADP and glucose-6-phosphate dehydrogenase to produce NADH or NADPH; and
a 2-3 process in which resazurin is reacted with the NADH or NADPH obtained in the 2-2 process and a diaphorase in the presence of an SH reagent, and the resulting fluorescence intensity or absorbance is measured.

**6.** The method for measuring ADP according to Claim 5,
wherein the SH reagent is a maleimide compound represented by the following General Formula [1] or 2-iodoacetamide,

[Chemical Formula 3]

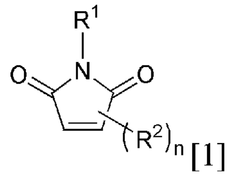

(where, in Formula [1], $R^1$, $R^2$, and n have the same meanings as described above).

**7.** The method for measuring ADP according to Claim 5,
wherein the maleimide compound represented by General Formula [1] is N-ethylmaleimide, maleimide or N-(2-sulfoethyl)maleimide.

**8.** A method for measuring activities of ADP-producing enzymes comprising:

a 1-1 process in which an ADP-producing enzyme is reacted with a substrate in the presence of ATP to convert the ATP into ADP;
a 2-1 process in which glucose is reacted with the ADP obtained in the 1-1 process and an ADP-dependent hexokinase to produce glucose-6-phosphate;
a 2-2 process in which the glucose-6-phosphate obtained in the 2-1 process is reacted with NAD or NADP and glucose-6-phosphate dehydrogenase to produce NADH or NADPH; and
a 2-3 process in which resazurin is reacted with the NADH or NADPH obtained in the 2-2 process and a diaphorase in the presence of an SH reagent, and the resulting fluorescence intensity or absorbance is measured.

**9.** The method for measuring activities of ADP-producing enzymes according to Claim 8,
wherein the SH reagent is a maleimide compound represented by the following General Formula [1] or 2-iodoacetamide,

[Chemical Formula 4]

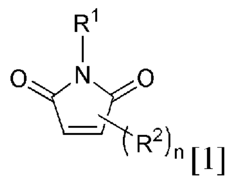

(where, in Formula [1], $R^1$, $R^2$, and n have the same meanings as described above).

**10.** The method for measuring activities ofADP-producing enzymes according to Claim 8,
wherein the maleimide compound represented by General Formula [1] is N-ethylmaleimide, maleimide or N-(2-sulfoethyl)maleimide.

**11.** The method for measuring activities of ADP-producing enzymes according to Claim 8,
wherein the ADP-producing enzyme is at least one type selected from the group including kinases, ATPases, nitrogenases, tetrahydrofolate synthases, acetyl-CoA carboxylase, pyruvate carboxylase, and glutathione synthase.

**12.** A method for measuring activities of a glycosyltransferase, the method comprising:

a first process in which GDP or UDP produced during a glycosyltransferase reaction is reacted with an NDP kinase in the presence of ATP, or CMP produced during the glycosyltransferase reaction is reacted with NMP kinase or a CMP kinase in the presence of ATP, and thus ADP corresponding to an amount of the GDP, UDP or CMP is produced;
a 2-1 process in which glucose is reacted with the ADP obtained in the first process and an ADP-dependent hexokinase to produce glucose-6-phosphate;
a 2-2 process in which the glucose-6-phosphate obtained in the 2-1 process is reacted with NAD or NADP and

glucose-6-phosphate dehydrogenase to produce NADH or NADPH; and
a 2-3 process in which reactions of the NADH or NADPH obtained in the 2-2 process and a diaphorase are caused in the presence of an SH reagent, and the resulting fluorescence intensity or absorbance is measured.

**13.** The method for measuring activities of a glycosyltransferase according to Claim 12,
wherein the SH reagent is a maleimide compound represented by the following General Formula [1] or 2-iodoacetamide,

[Chemical Formula 5]

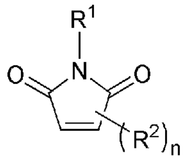

[1]

(where, in Formula [1], $R^1$, $R^2$, and n have the same meanings as described above).

**14.** The method for measuring activities of a glycosyltransferase according to Claim 12,
wherein the maleimide compound represented by General Formula [1] is N-ethylmaleimide, maleimide or N-(2-sulfoethyl)maleimide.

**15.** The method for measuring activities of a glycosyltransferase according to Claim 12,
wherein the glycosyltransferase is at least one type selected from the group including fucosyltransferases, mannosyltransferases, glucosyltransferases, galactosyltransferases, glucuronosyltransferases, xylosyltransferases, N-acetylglucosaminyltransferases, N-acetylgalactosaminyltransferases, and sialyltransferases.

**16.** An ADP measurement kit comprising glucose, an ADP-dependent hexokinase, glucose-6-phosphate dehydrogenase, a diaphorase, NAD and/or NADP, resazurin and an SH reagent.

**17.** The ADP measurement kit according to Claim 16,
wherein the SH reagent is a maleimide compound represented by the following General Formula [1] or 2-iodoacetamide,

[Chemical Formula 6]

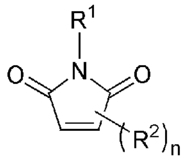

[1]

(where, in Formula [1], $R^1$, $R^2$, and n have the same meanings as described above).

**18.** The ADP measurement kit according to Claim 16,
wherein the maleimide compound represented by General Formula [1] is N-ethylmaleimide, maleimide or N-(2-sulfoethyl)maleimide.

**19.** An ADP-producing enzyme activity measurement kit comprising glucose, an ADP-dependent hexokinase, glucose-6-phosphate dehydrogenase, a diaphorase, NAD and/or NADP, resazurin and an SH reagent.

**20.** The ADP-producing enzyme activity measurement kit according to Claim 19, wherein the SH reagent is a maleimide compound represented by the following General Formula [1] or 2-iodoacetamide,

[Chemical Formula 7]

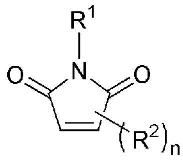

[1]

(where, in Formula [1], $R^1$, $R^2$, and n have the same meanings as described above).

**21.** The ADP-producing enzyme activity measurement kit according to Claim 20, wherein the maleimide compound represented by General Formula [1] is N-ethylmaleimide, maleimide or N-(2-sulfoethyl)maleimide.

**22.** A glycosyltransferase activity measurement kit comprising:

a first solution including ATP and NMP kinase, an NDP kinase or a CMP kinase; and
a second solution including glucose, an ADP-dependent hexokinase, glucose-6-phosphate dehydrogenase, a diaphorase, NAD and/or NADP, resazurin, and an SH reagent.

**23.** The glycosyltransferase activity measurement kit according to Claim 22, wherein the first solution further includes a reducing agent.

**24.** The glycosyltransferase activity measurement kit according to Claim 22, wherein the SH reagent is a maleimide compound represented by the following General Formula [1] or 2-iodoacetamide,

[Chemical Formula 8]

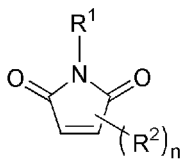

[1]

(where, in Formula [1], $R^1$, $R^2$, and n have the same meanings as described above).

**25.** The glycosyltransferase activity measurement kit according to Claim 24, wherein the maleimide compound represented by General Formula [1] is N-ethylmaleimide, maleimide or N-(2-sulfoethyl)maleimide.

FIG. 1

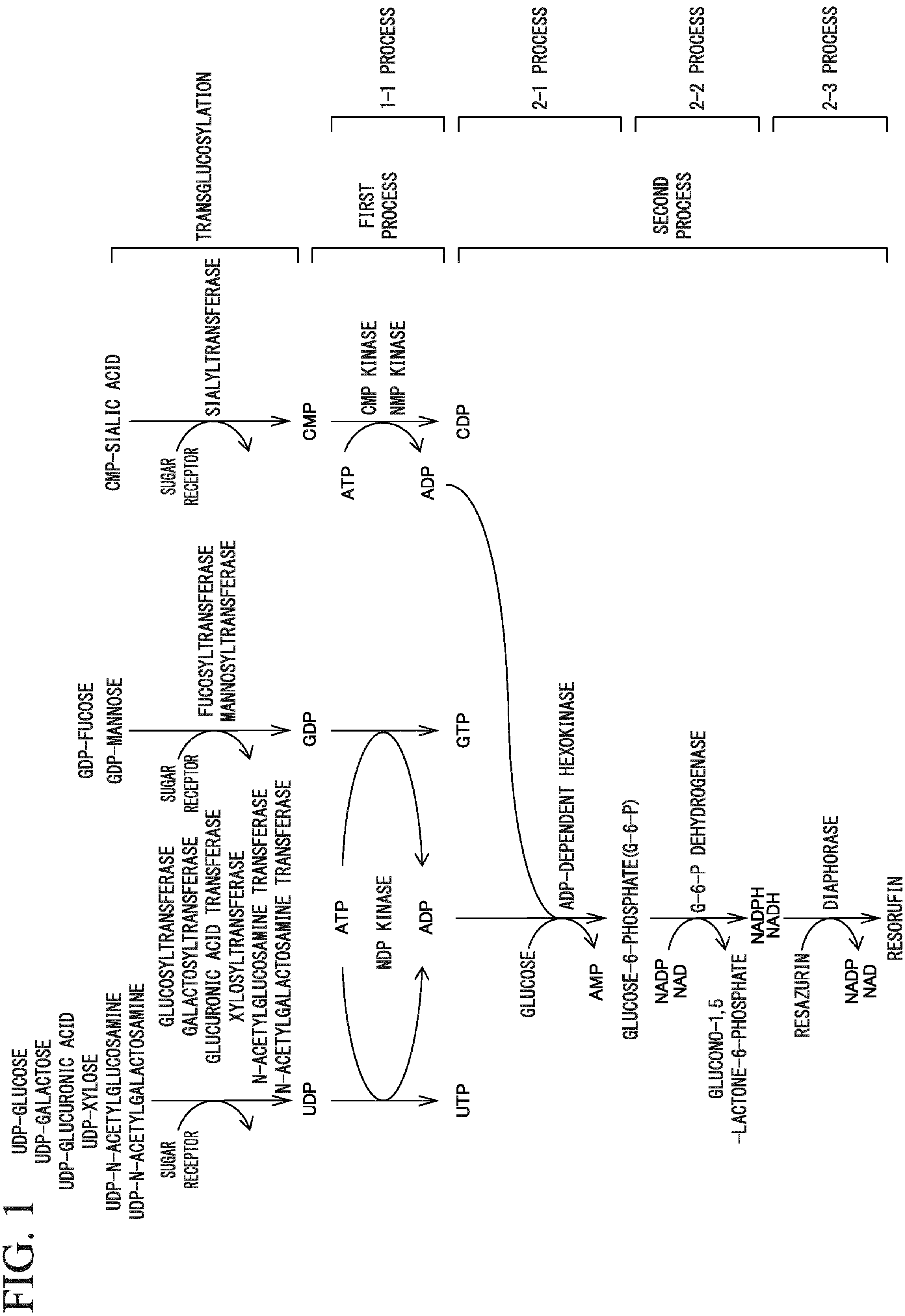

TRANSGLUCOSYLATION
FIRST PROCESS
SECOND PROCESS
1-1 PROCESS
2-1 PROCESS
2-2 PROCESS
2-3 PROCESS
CMP-SIALIC ACID
SUGAR RECEPTOR
SIALYLTRANSFERASE
CMP
ATP
CMP KINASE
NMP KINASE
ADP
CDP
GDP-FUCOSE
GDP-MANNOSE
SUGAR RECEPTOR
FUCOSYLTRANSFERASE
MANNOSYLTRANSFERASE
GDP
GTP
UDP-GLUCOSE
UDP-GALACTOSE
UDP-GLUCURONIC ACID
UDP-XYLOSE
UDP-N-ACETYLGLUCOSAMINE
UDP-N-ACETYLGALACTOSAMINE
SUGAR RECEPTOR
GLUCOSYLTRANSFERASE
GALACTOSYLTRANSFERASE
GLUCURONIC ACID TRANSFERASE
XYLOSYLTRANSFERASE
N-ACETYLGLUCOSAMINE TRANSFERASE
N-ACETYLGALACTOSAMINE TRANSFERASE
UDP
UTP
ATP
NDP KINASE
ADP
GLUCOSE
ADP-DEPENDENT HEXOKINASE
AMP
GLUCOSE-6-PHOSPHATE(G-6-P)
NADP
NAD
G-6-P DEHYDROGENASE
GLUCONO-1,5
-LACTONE-6-PHOSPHATE
NADPH
NADH
RESAZURIN
DIAPHORASE
NADP
NAD
RESORUFIN

FIG. 2

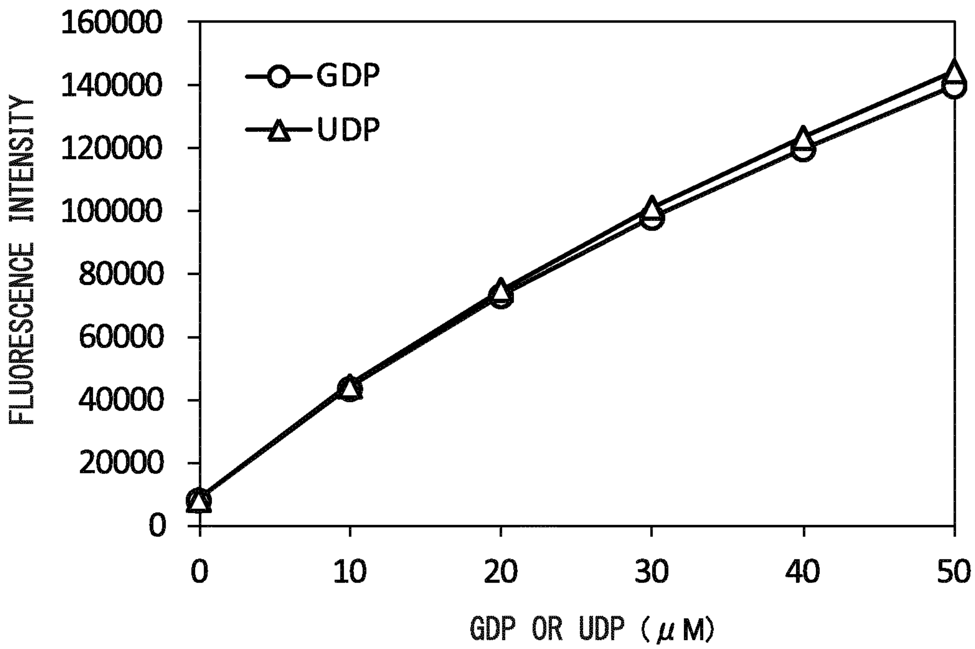
FLUORESCENCE INTENSITY
GDP
UDP
GDP OR UDP (μM)
0
20000
40000
60000
80000
100000
120000
140000
160000
0
10
20
30
40
50

FIG. 3

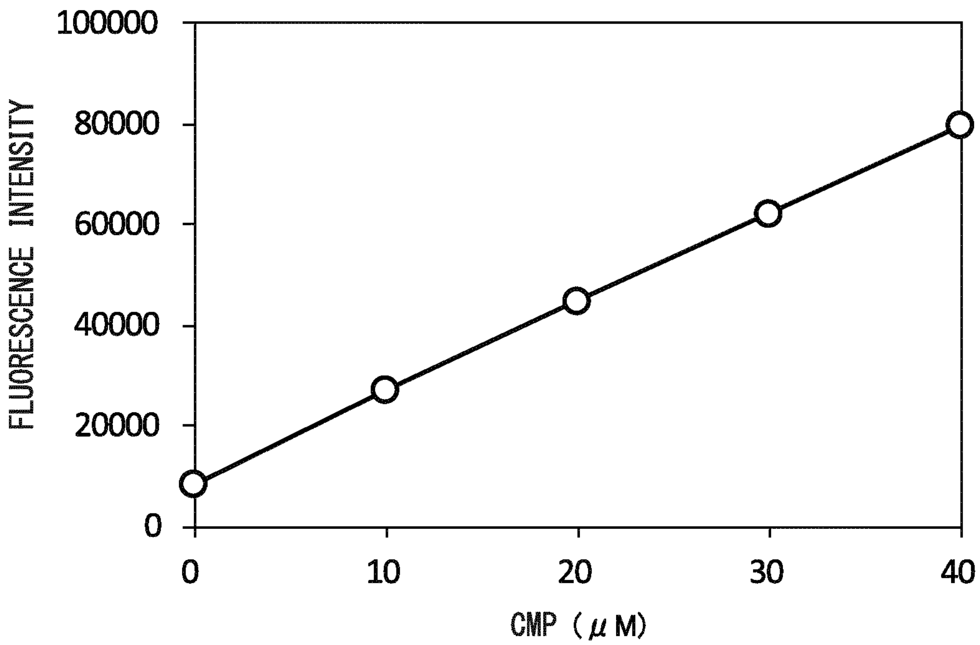
FLUORESCENCE INTENSITY
CMP (μM)
0
20000
40000
60000
80000
100000
0
10
20
30
40

FIG. 4

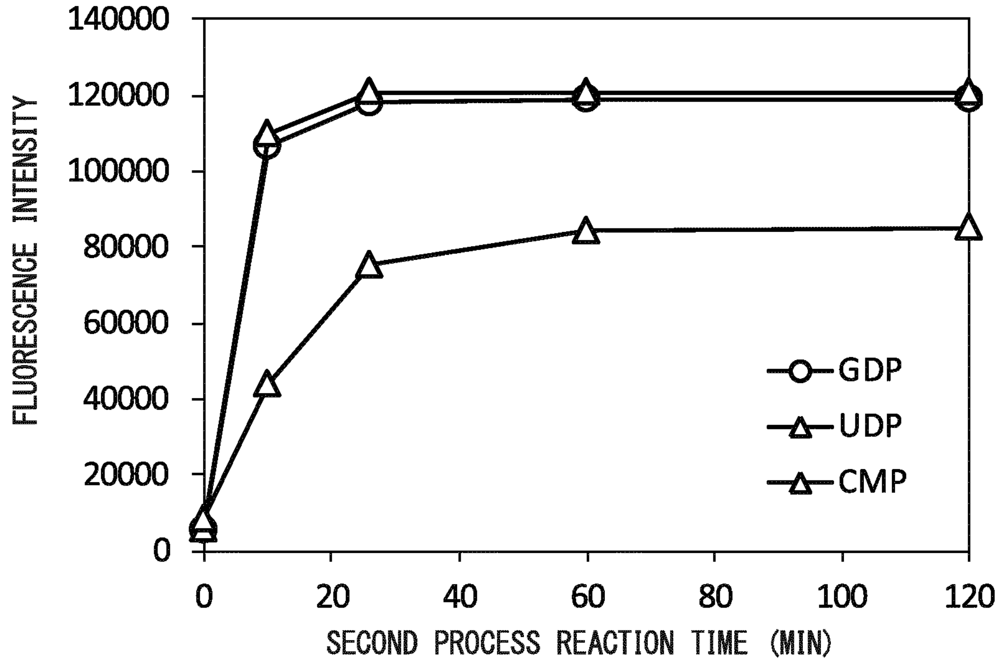
140000
120000
100000
80000
60000
40000
20000
0
FLUORESCENCE INTENSITY
0
20
40
60
80
100
120
SECOND PROCESS REACTION TIME (MIN)
GDP
UDP
CMP

FIG. 5

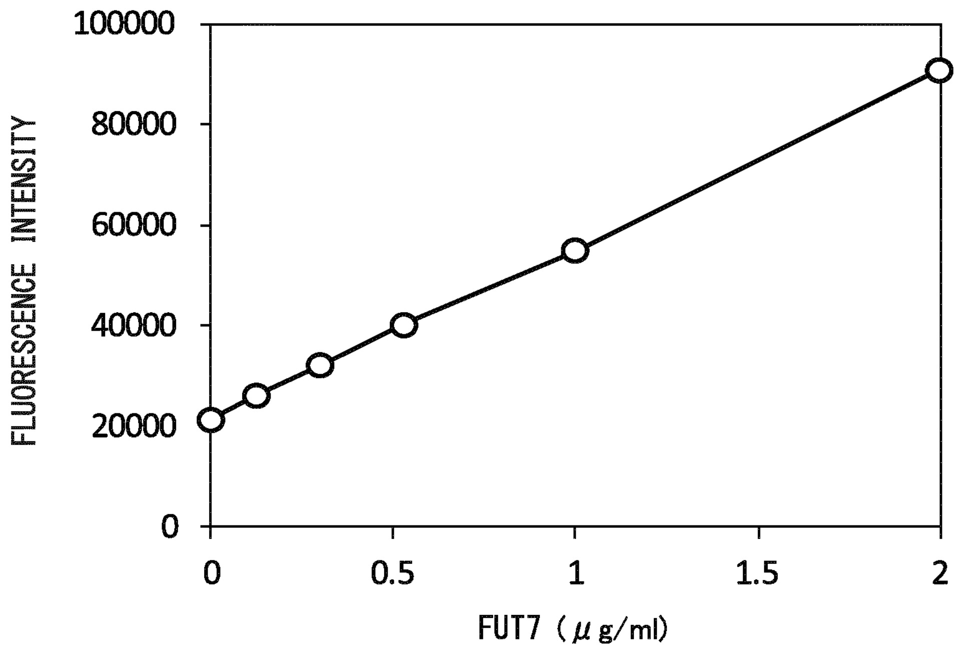
100000
80000
60000
40000
20000
0
FLUORESCENCE INTENSITY
0
0.5
1
1.5
2
FUT7 (μg/ml)

FIG. 6

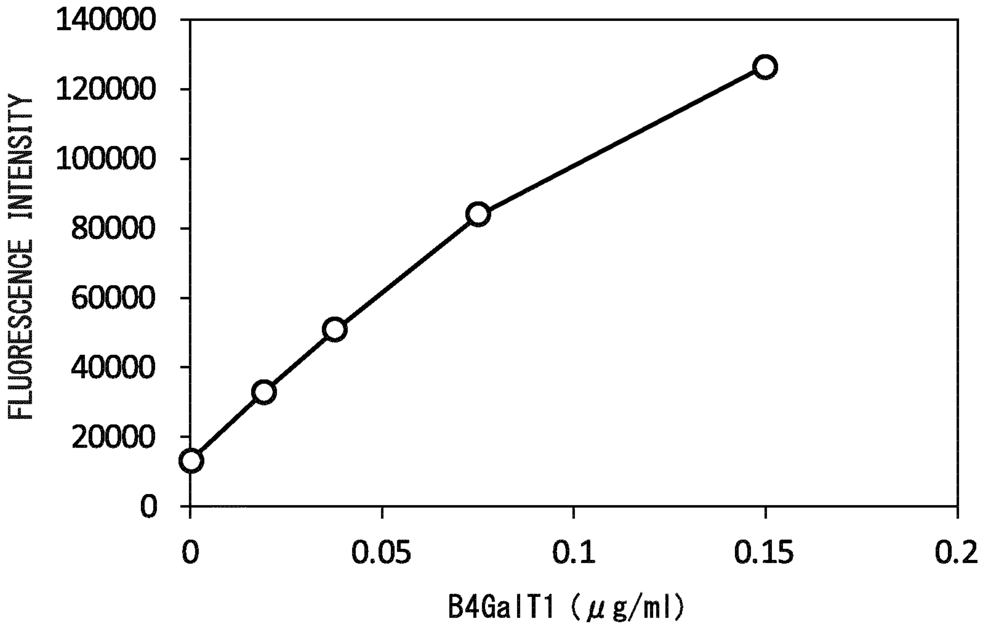
FLUORESCENCE INTENSITY
140000
120000
100000
80000
60000
40000
20000
0
0
0.05
0.1
0.15
0.2
B4GalT1 (μg/ml)

FIG. 7

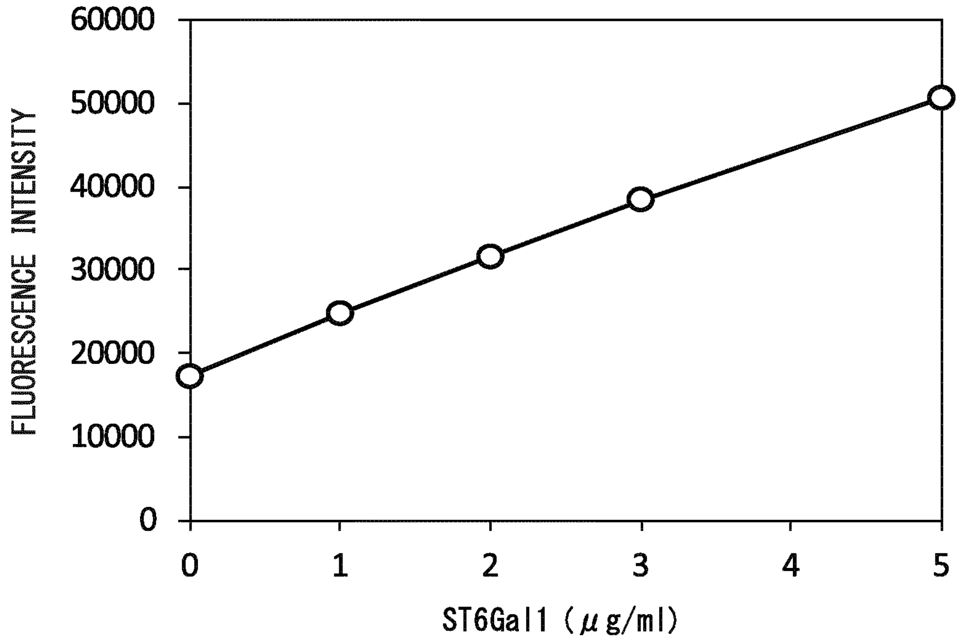
FLUORESCENCE INTENSITY
60000
50000
40000
30000
20000
10000
0
0
1
2
3
4
5
ST6Gal1 (μg/ml)

FIG. 8

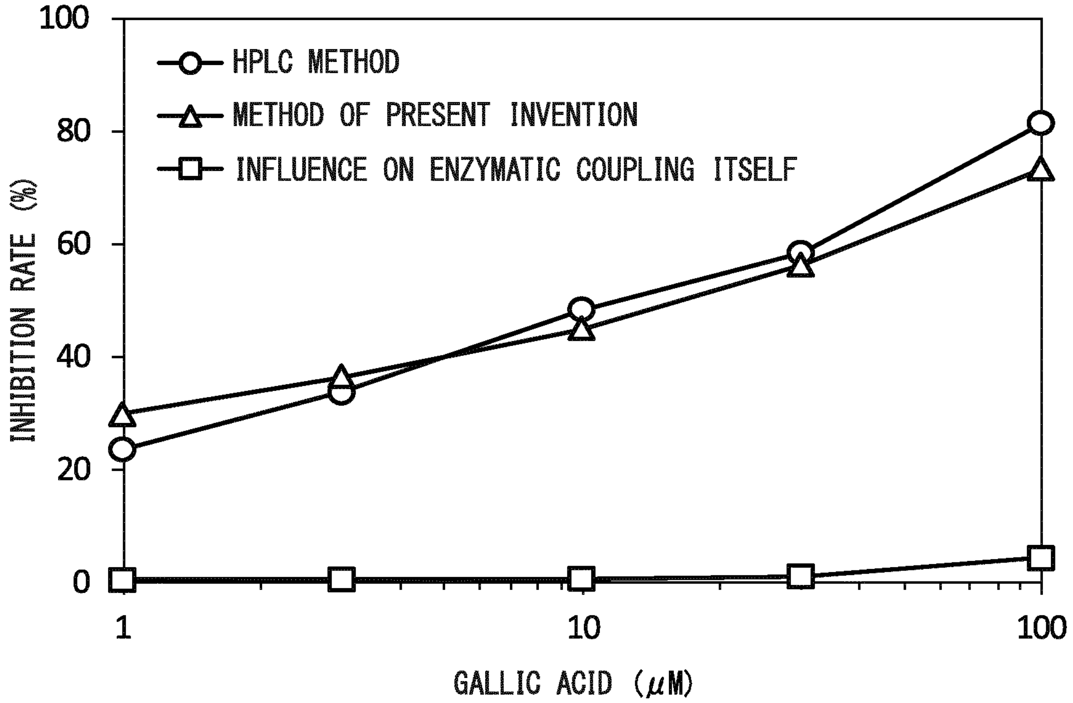
100
80
60
40
20
0
INHIBITION RATE (%)
HPLC METHOD
METHOD OF PRESENT INVENTION
INFLUENCE ON ENZYMATIC COUPLING ITSELF
1
10
100
GALLIC ACID (μM)

FIG. 9

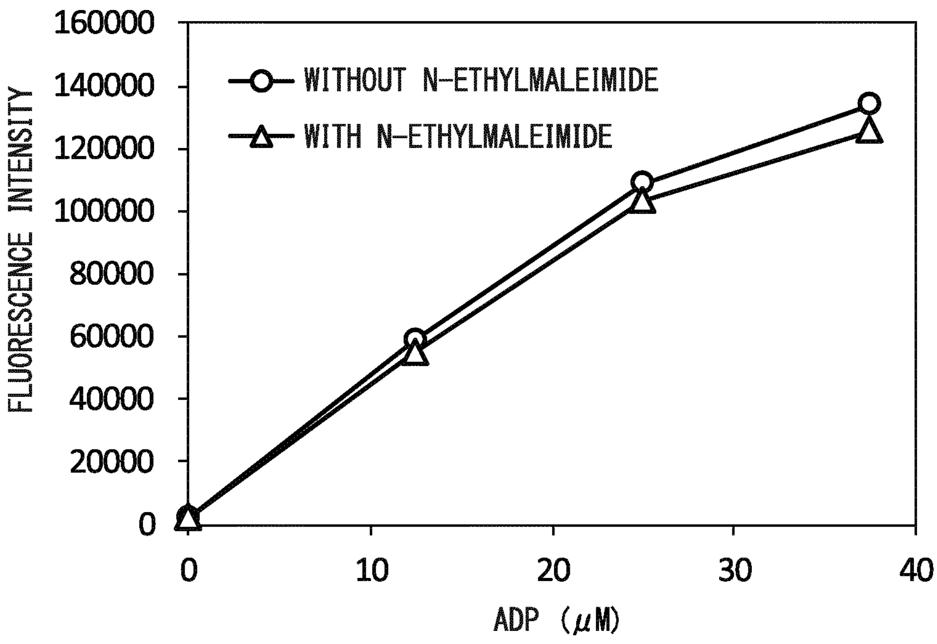
160000
140000
120000
100000
80000
60000
40000
20000
0
FLUORESCENCE INTENSITY
WITHOUT N-ETHYLMALEIMIDE
WITH N-ETHYLMALEIMIDE
0
10
20
30
40
ADP (μM)

FIG. 10

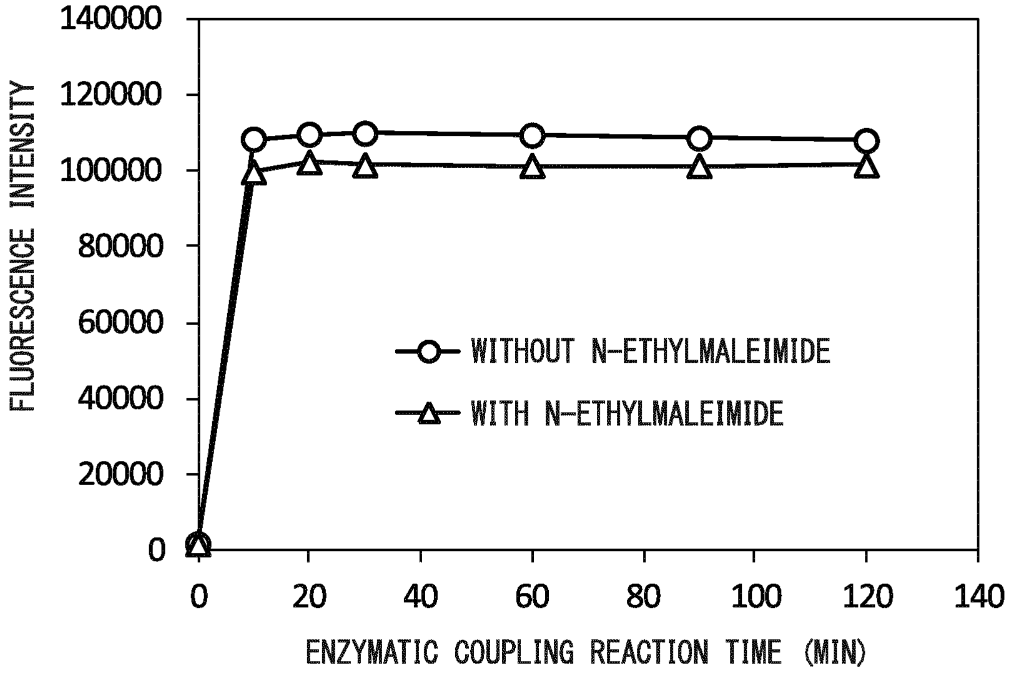
140000
120000
100000
80000
60000
40000
20000
0
FLUORESCENCE INTENSITY
0
20
40
60
80
100
120
140
ENZYMATIC COUPLING REACTION TIME (MIN)
WITHOUT N-ETHYLMALEIMIDE
WITH N-ETHYLMALEIMIDE

FIG. 11

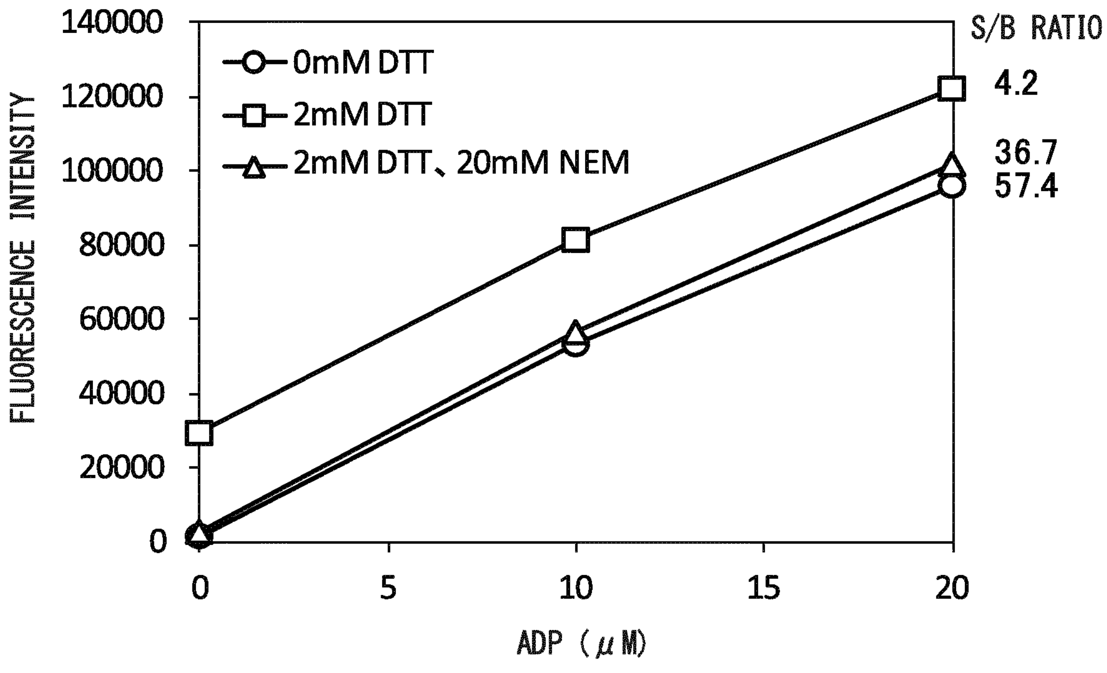
140000
120000
100000
80000
60000
40000
20000
0
FLUORESCENCE INTENSITY
0
5
10
15
20
ADP (μM)
0mM DTT
2mM DTT
2mM DTT、20mM NEM
S/B RATIO
4.2
36.7
57.4

FIG. 12

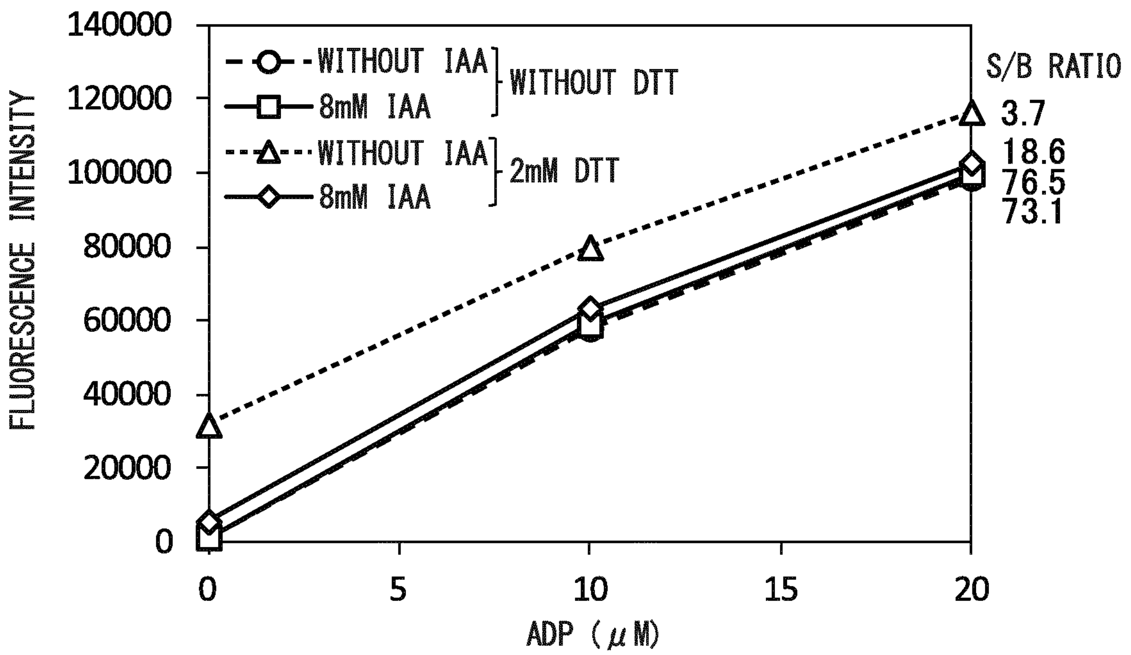
140000
120000
100000
80000
60000
40000
20000
0
FLUORESCENCE INTENSITY
WITHOUT IAA
8mM IAA
WITHOUT DTT
WITHOUT IAA
8mM IAA
2mM DTT
S/B RATIO
3.7
18.6
76.5
73.1
0
5
10
15
20
ADP (μM)

FIG. 13

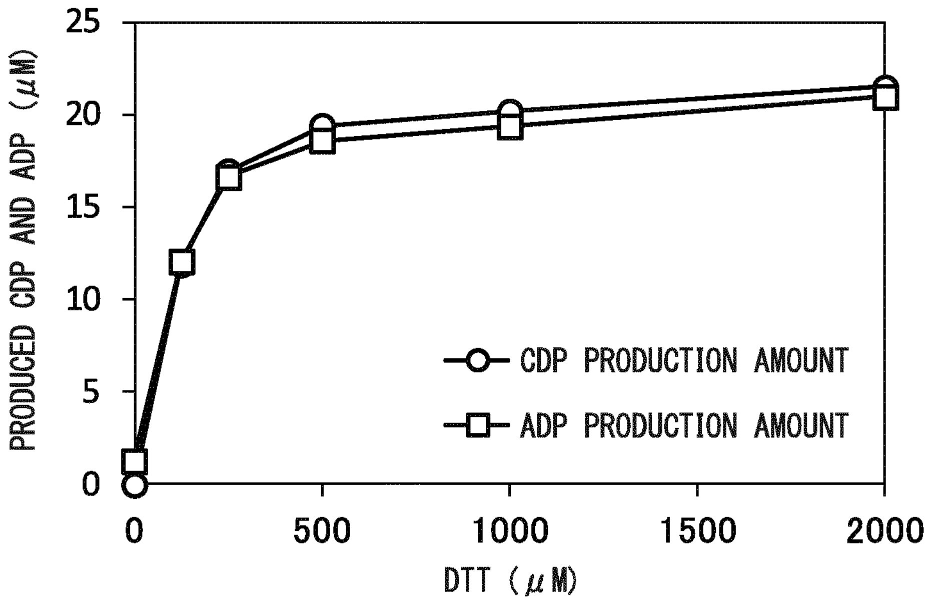
25
20
15
10
5
0
PRODUCED CDP AND ADP (μM)
CDP PRODUCTION AMOUNT
ADP PRODUCTION AMOUNT
0
500
1000
1500
2000
DTT (μM)

FIG. 14

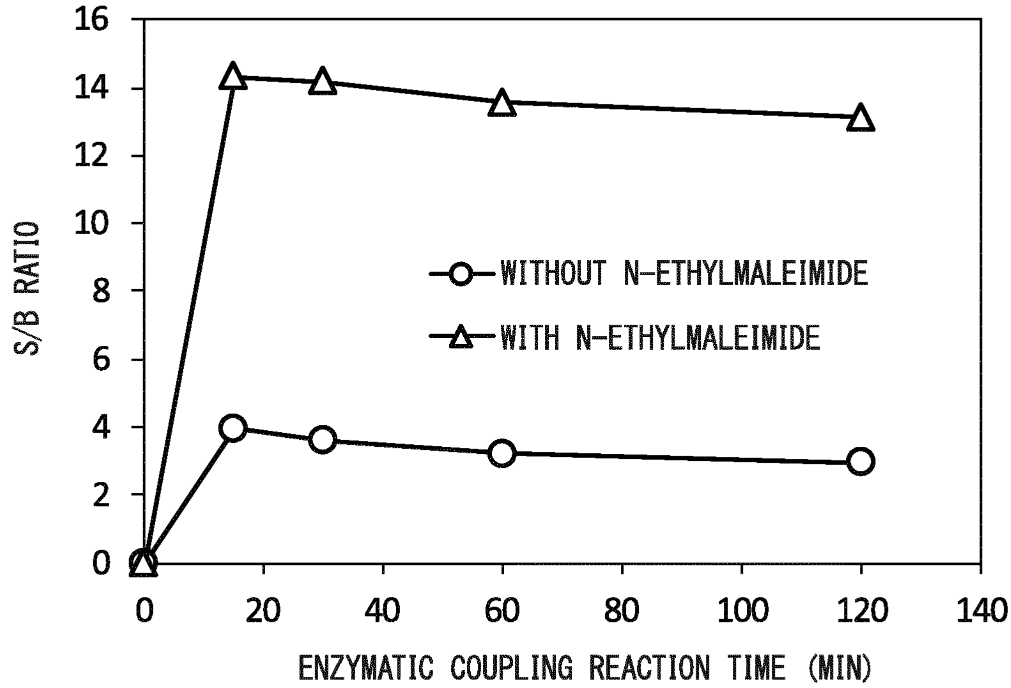
16
14
12
10
8
6
4
2
0
S/B RATIO
WITHOUT N-ETHYLMALEIMIDE
WITH N-ETHYLMALEIMIDE
0
20
40
60
80
100
120
140
ENZYMATIC COUPLING REACTION TIME (MIN)

FIG. 15

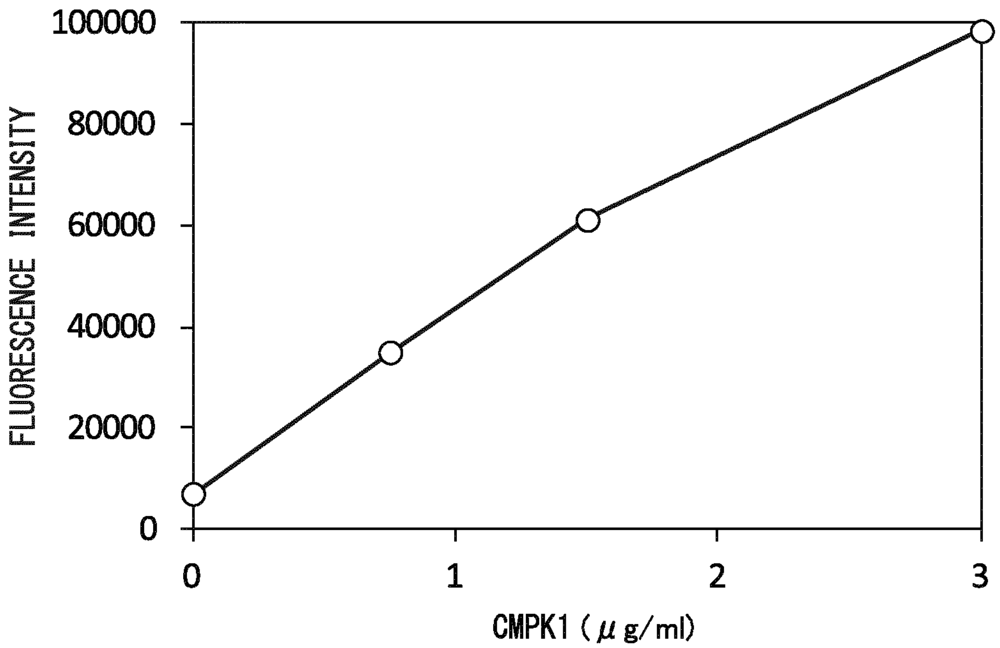
100000
80000
60000
40000
20000
0
FLUORESCENCE INTENSITY
0
1
2
3
CMPK1 (μg/ml)

FIG. 16

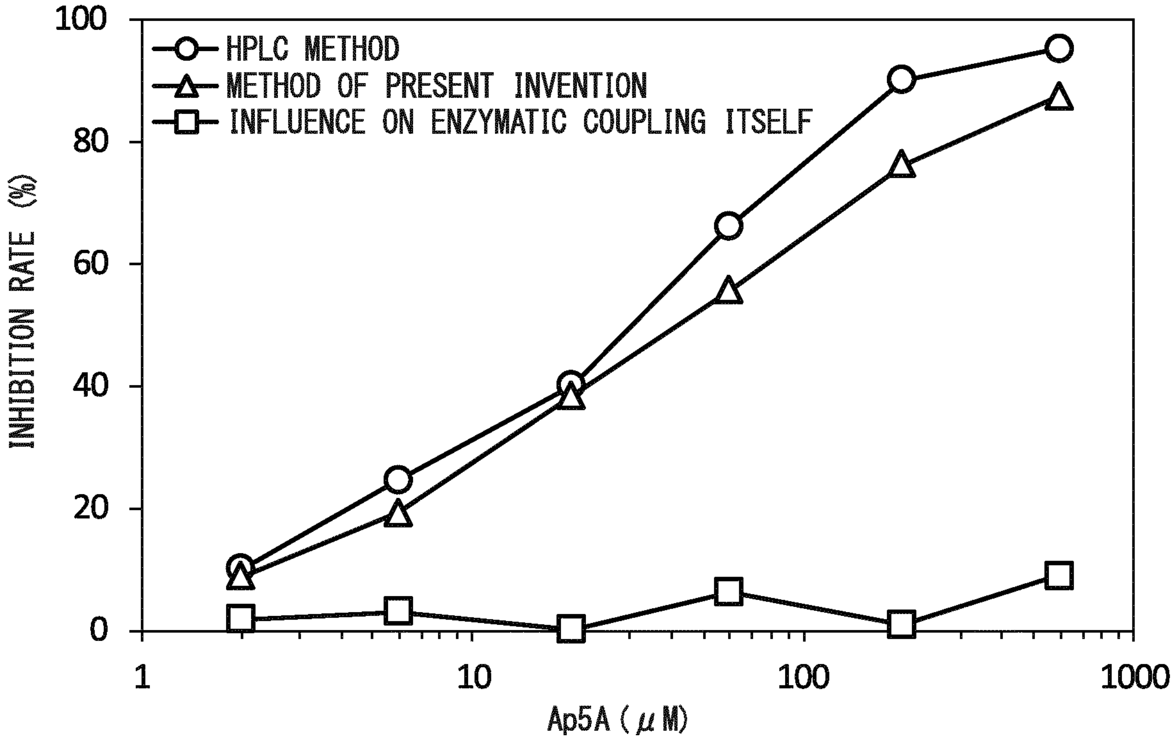
100
80
60
40
20
0
INHIBITION RATE (%)
HPLC METHOD
METHOD OF PRESENT INVENTION
INFLUENCE ON ENZYMATIC COUPLING ITSELF
1
10
100
1000
Ap5A (μM)

# FIG. 17A

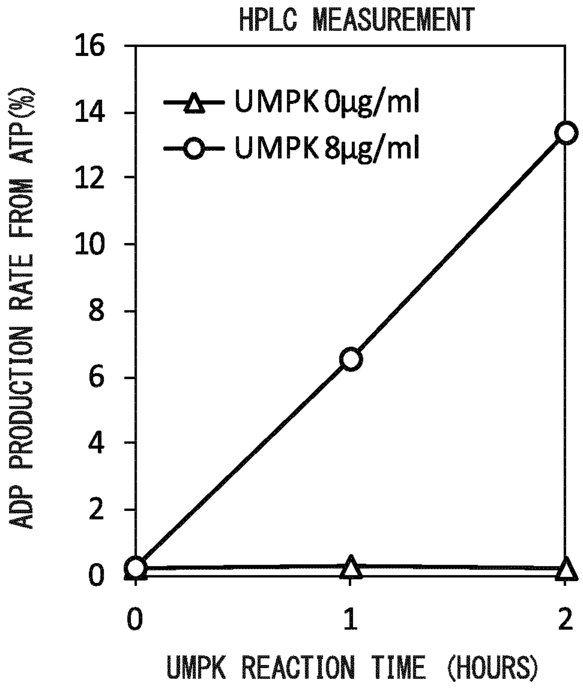


# FIG. 17B

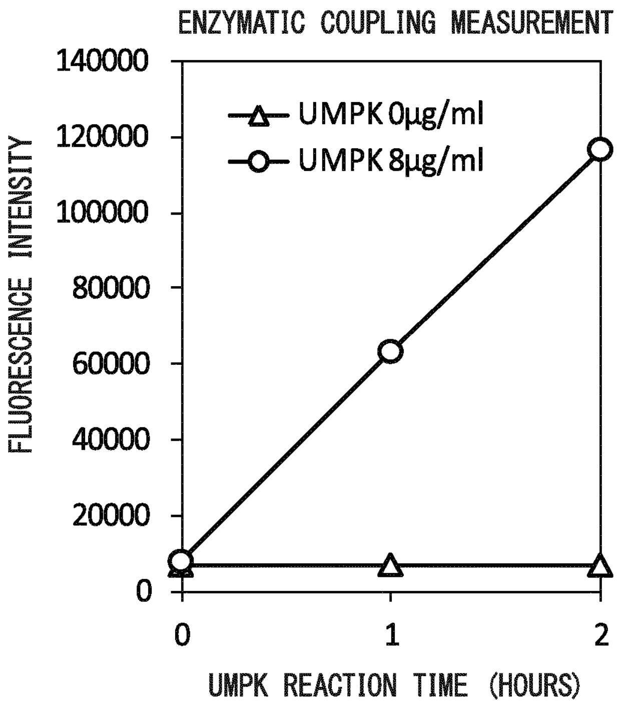

FIG. 18A

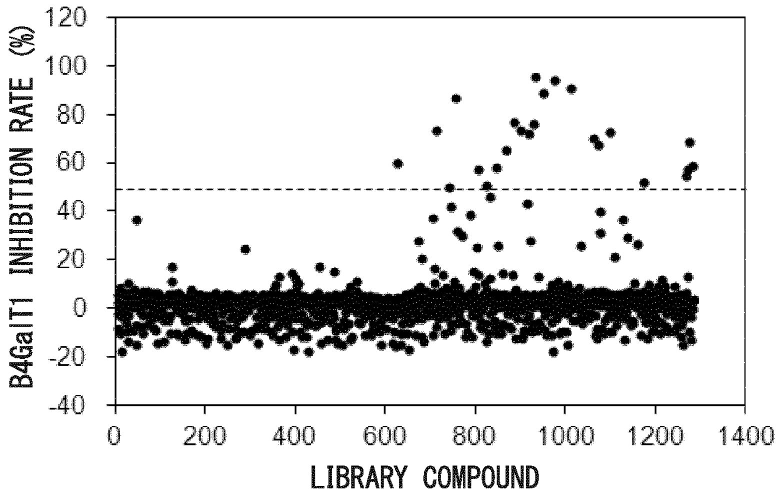


FIG. 18B

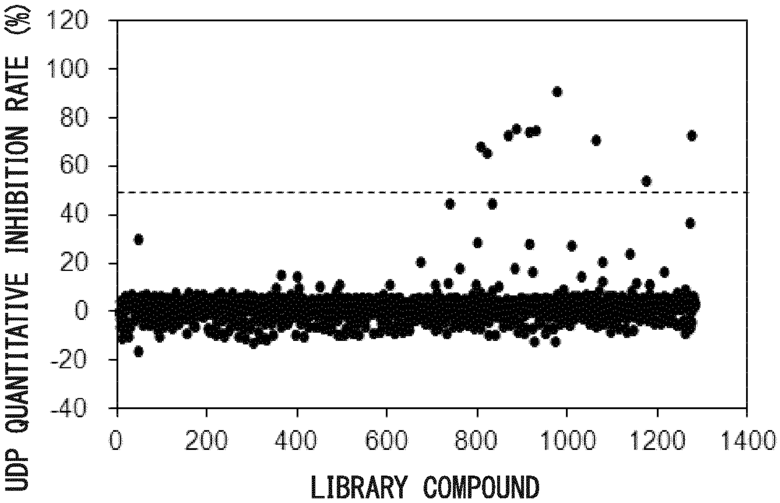


FIG. 18C

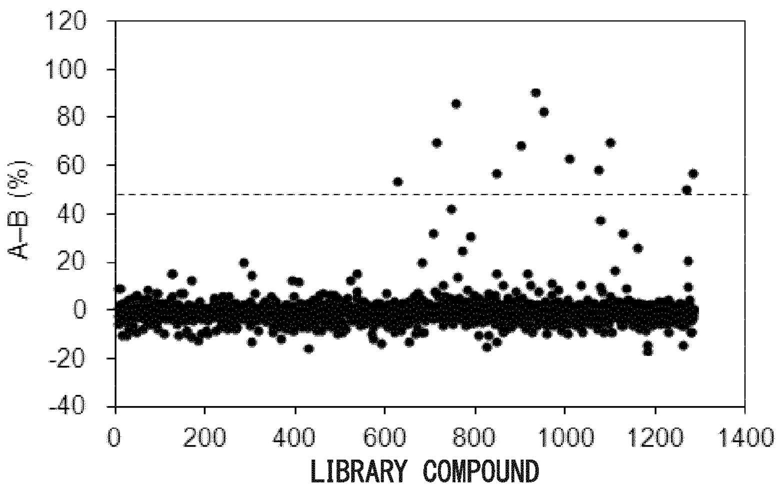

FIG. 19A

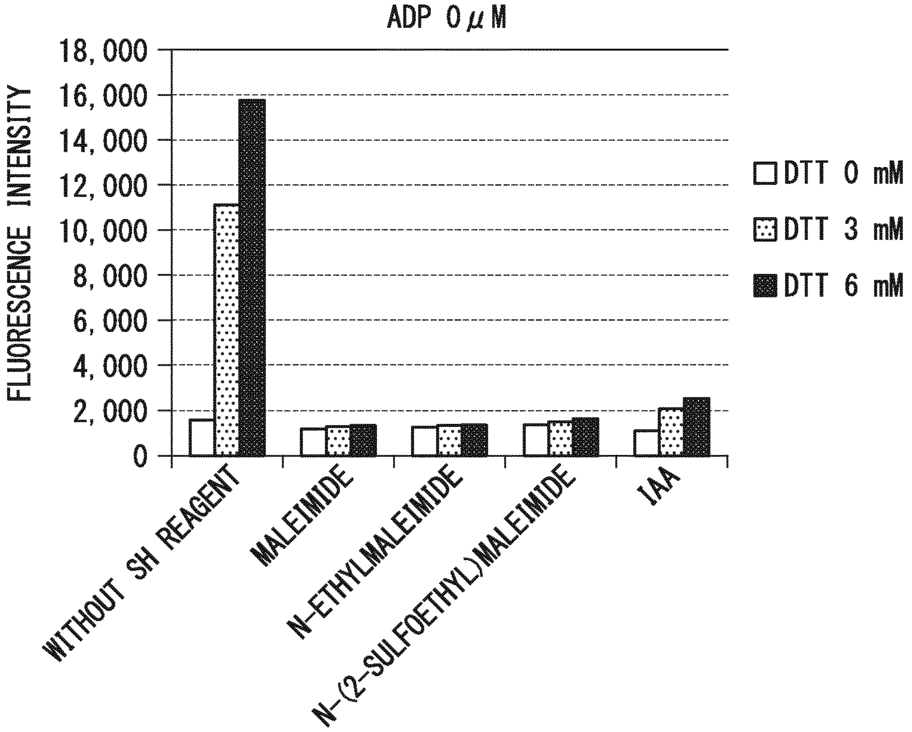


FIG. 19B

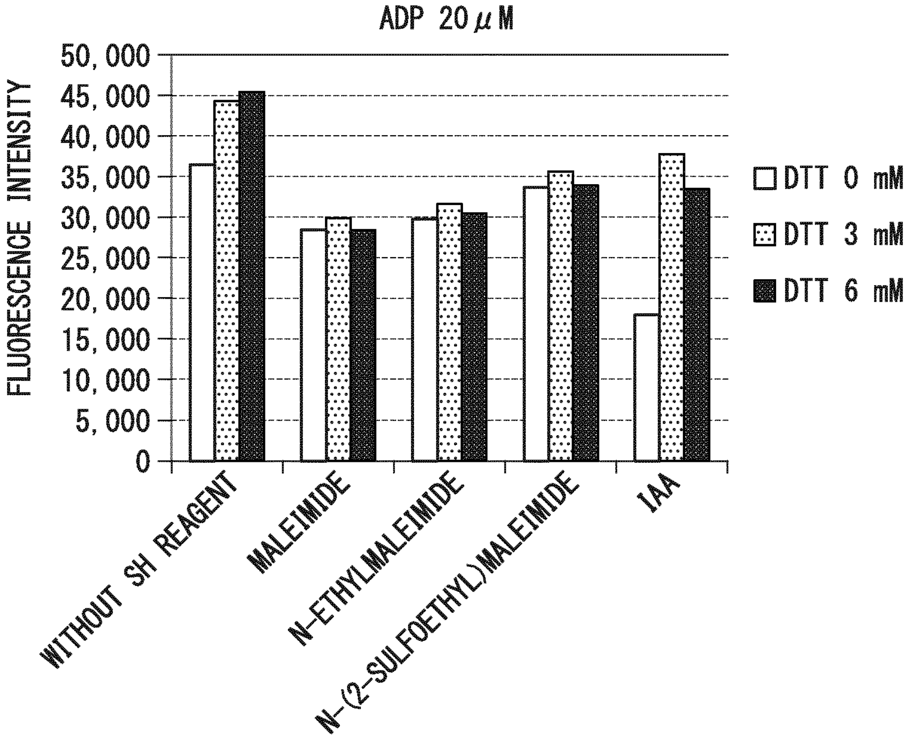

# FIG. 19C

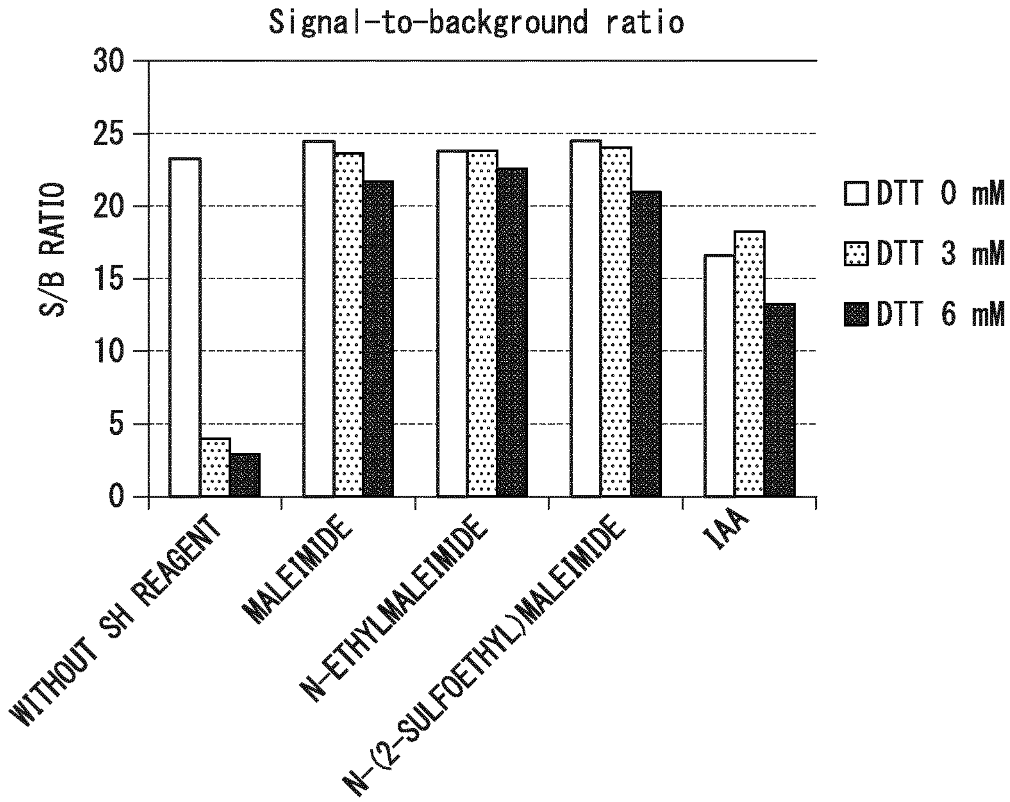
Signal-to-background ratio
30
25
20
15
10
5
0
S/B RATIO
DTT 0 mM
DTT 3 mM
DTT 6 mM
WITHOUT SH REAGENT
MALEIMIDE
N-ETHYLMALEIMIDE
N-(2-SULFOETHYL)MALEIMIDE
IAA

FIG. 20A

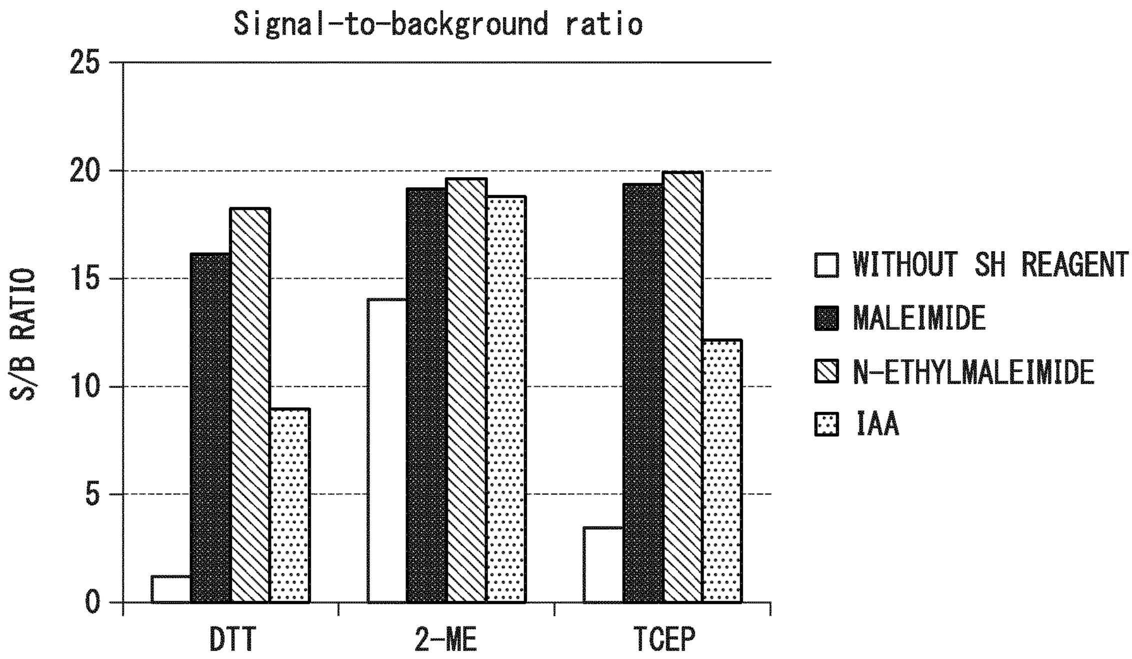


FIG. 20B

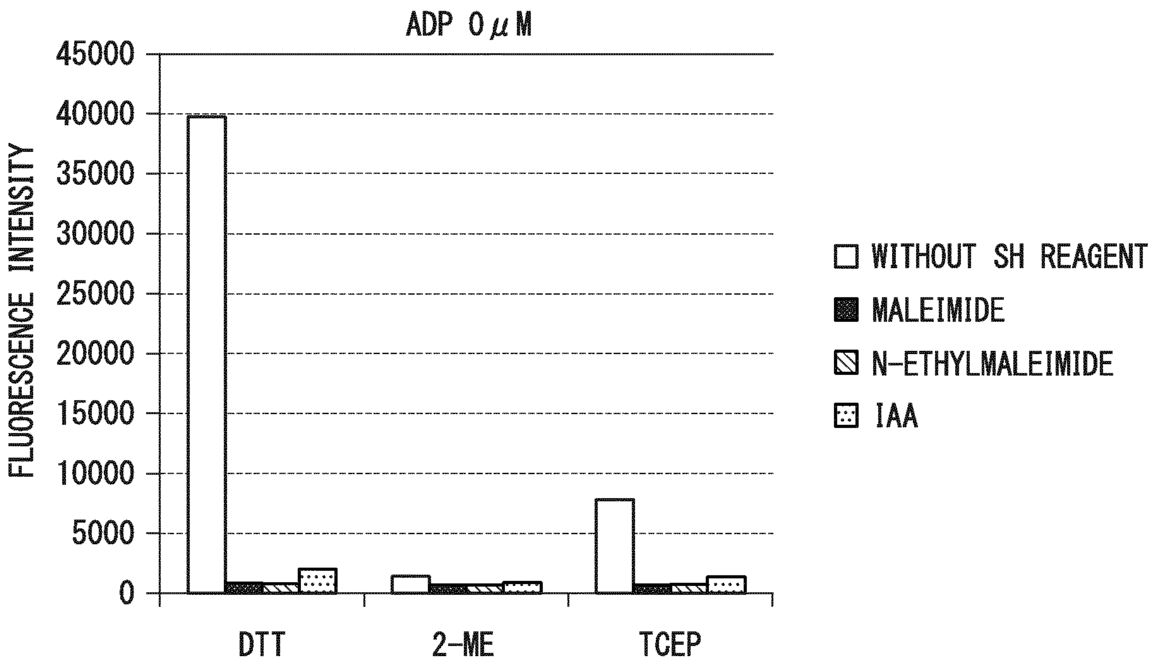

FIG. 20C

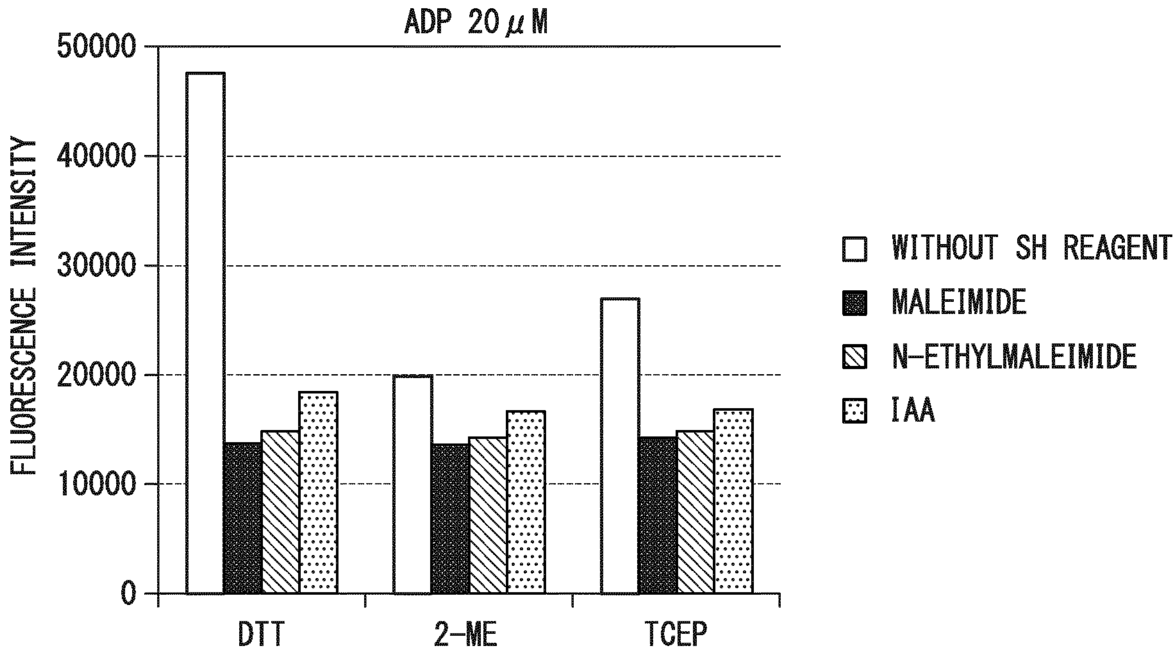
ADP 20 μM
FLUORESCENCE INTENSITY
50000
40000
30000
20000
10000
0
DTT
2-ME
TCEP
WITHOUT SH REAGENT
MALEIMIDE
N-ETHYLMALEIMIDE
IAA

## INTERNATIONAL SEARCH REPORT

International application No.
PCT/JP2014/080865

A. CLASSIFICATION OF SUBJECT MATTER
*C12Q1/26*(2006.01)i, *C12Q1/32*(2006.01)i, *C12Q1/48*(2006.01)i, *G01N21/78* (2006.01)i

According to International Patent Classification (IPC) or to both national classification and IPC

B. FIELDS SEARCHED

Minimum documentation searched (classification system followed by classification symbols)
C12Q1/26, C12Q1/32, C12Q1/48, G01N21/78

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/MEDLINE/BIOSIS(STN), JSTPlus/JMEDPlus/JST7580(JDreamIII), Thomson Innovation

C. DOCUMENTS CONSIDERED TO BE RELEVANT

| Category* | Citation of document, with indication, where appropriate, of the relevant passages | Relevant to claim No. |
|---|---|---|
| A | JP 2005-021025 A (Takeda Food Products, Ltd.), 27 January 2005 (27.01.2005), claims; paragraphs [0021] to [0022]; examples (Family: none) | 1-25 |
| A | JP 05-207895 A (Arinobu FUJIMURA), 20 August 1993 (20.08.1993), claims; paragraphs [0010] to [0014] (Family: none) | 1-25 |
| A | JP 62-239999 A (Arinobu FUJIMURA), 20 October 1987 (20.10.1987), claims; page 2, upper right column, line 6 to lower right column, line 17 (Family: none) | 1-25 |

☒ Further documents are listed in the continuation of Box C.  ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 06 February 2015 (06.02.15) | 17 February 2015 (17.02.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | <br><br>Telephone No. |

Form PCT/ISA/210 (second sheet) (July 2009)

**INTERNATIONAL SEARCH REPORT**

International application No.
PCT/JP2014/080865

C (Continuation). DOCUMENTS CONSIDERED TO BE RELEVANT

| Category* | Citation of document, with indication, where appropriate, of the relevant passages | Relevant to claim No. |
|---|---|---|
| A | Takuji SHODA et al., "Kakushu Maleimide-ki o Yusuru Keiko Shikiso no Hannosei to Kogaku Tokusei", Abstracts of Annual Meeting of Pharmaceutical Society of Japan, 2006, vol.126, no.2, page 42 | 1-25 |
| A | Takuya MATSUMOTO et al., "A Novel Fluorescence Probe for Thiol Modification", Abstracts of Annual Meeting of Pharmaceutical Society of Japan, 2008, vol.128, no.4, page 7 | 1-25 |
| A | JP 2005-500406 A (Amersham Biosciences UK Ltd.), 06 January 2005 (06.01.2005), claims; paragraph [0007]; table 1 & US 2004/0191792 A1 & EP 1392776 A2 & WO 2002/099424 A2 & GB 2391870 A | 1-25 |
| A | JP 2005-501228 A (Amersham Biosciences UK Ltd.), 13 January 2005 (13.01.2005), claims; paragraph [0005]; table 1 & US 2004/171014 A1 & EP 1399742 A2 & WO 2002/0099432 A2 & GB 2391550 A | 1-25 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013240926 A
- JP 2005046029 A
- JP HEI9234098 B
- JP 2011103825 A
- US 7338775 B


### Non-patent literature cited in the description


- Dictionary of Biochemistry. Tokyo Kagaku Dojin, 2007, 931
- Essentials of Glycobiology. Cold Spring Harbor Laboratory Press, 2009
- Sugar chain engineering can be seen properly. Institute of Advanced Industrial Science and Technology. Hakujitsusha, 2008
- *ChemBioChem,* 2010, vol. 11 (14), 1939-1949
- *Anal. Biochem,* 1980, vol. 102 (2), 441-449
- *Anal. Biochem,* 1994, vol. 220 (1), 92-97
- *Glycobiology,* 2011, vol. 21 (6), 727-733
- *Angew. Chem. Int. Ed.,* 2011, vol. 50 (52), 12534-12537
- Dictionary of Biochemistry. Tokyo Kagaku Dojin, 2007, 340
- New challenge for molecularly targeted drug development, drug discovery story of potential molecularly targeted drug and new drug development trend to next-generation drug discovery technology. Experimental Medicine. Yodosha, 2009, vol. 27
- Dictionary of Biochemistry. Tokyo Kagaku Dojin, 2007, 173
- *Arch. Biochem. Biophys,* 2004, vol. 425 (1), 51-57
- *J. Biol. Chem,* 1990, vol. 265 (11), 6339-6345